(19) Europäisches Patentamt
European Patent Office
Office européen des brevets

(11) Publication number: 0 083 186
B1

(12) **EUROPEAN PATENT SPECIFICATION**

(45) Date of publication of patent specification: **11.05.88**

(51) Int. Cl.$^4$: **C 07 D 417/12,** C 07 D 417/14, A 61 K 31/505

(21) Application number: **82306785.5**

(22) Date of filing: **20.12.82**

(54) **Thiazole derivatives as H2-receptor antagonists.**

(30) Priority: **28.12.81 US 334784**

(43) Date of publication of application:
**06.07.83 Bulletin 83/27**

(45) Publication of the grant of the patent:
**11.05.88 Bulletin 88/19**

(84) Designated Contracting States:
**AT BE CH DE FR GB IT LI LU NL SE**

(56) References cited:
**EP-A-0 015 138**
**EP-A-0 017 679**
**EP-A-0 049 618**
**DE-A-2 643 670**
**FR-A-2 229 417**
**US-A-4 200 578**

(73) Proprietor: **ELI LILLY AND COMPANY**
**307, East McCarty Street**
**Indianapolis Indiana 46285 (US)**

(72) Inventor: **Pioch, Richard Paul**
**3750, Briarwood Drive**
**Indianapolis Indiana 46240 (US)**

(74) Representative: **Crowther, Terence Roger et al**
**Erl Wood Manor**
**Windlesham Surrey GU20 6PH (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 083 186 B1

**Description**

This invention relates to $H_2$-receptor antagonists useful in treating peptic ulcers. More particularly the invention is concerned with novel pyrimidine derivatives having such properties.

The most successful anti-ulcer drug heretofore developed is cimetidine:

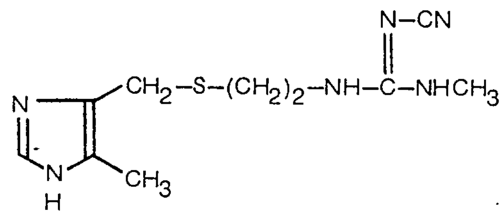

see, for instance, U.S. Patent Specification No. 3,950,333 and *J. Int. Med. Res., 3,* 86 (1975). There can be no question that the advent of cimetidine has constituted a major advance in the treatment of peptic ulcers. Indeed, the total sales of this drug are now well in excess of one billion dollars. However, there have recently appeared in the medical press a number of articles concerning possible untoward side-effects exhibited by cimetidine. Thus, there exists a continuing need to discover new and effective therapeutic agents in this area having minimal side-effects.

Pyrimidine derivatives having histamine $H_2$-antagonist activity are known from EP—A—0 015 138.

In accordance with the invention it has been discovered that pyrimidine derivatives of formula XX

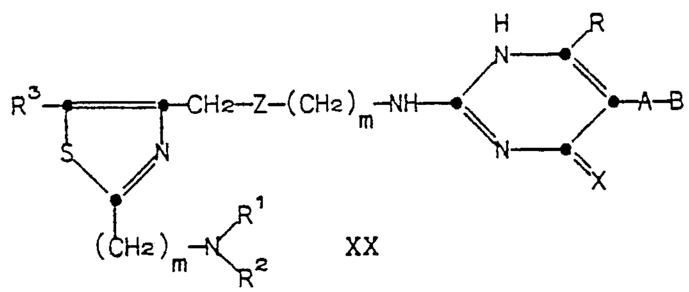

XX

wherein

R and $R^3$ independently represent hydrogen or $(C_1—C_3)$alkyl;

$R^1$ represents methyl;

$R^2$ represents methyl;

X is S or O;

Z is O, S or $CH_2$;

n is 2 or 3 when Z is O or S and n is 1, 2 or 3 when Z is $CH_2$;

$R^5$ is H;

m is 1;

A is $(C_1—C_5)$alkylene or $(CH_2)_qX(CH_2)_p$ wherein q and p are individually 0, 1, 2 or 3 and the sum of q plus p is 0—4, and

B is H, $CH_3$, $(C_3—C_6)$cycloalkyl, a pyridyl group optionally substituted with one or two $(C_1—C_3)$alkyl, $(C_1—C_3)$alkoxy, halo, hydroxy or amino groups; naphthyl; 1,3-benzodioxolyl; 2,3-dihydro-1,4-benzodioxinyl; phenyl optionally substituted with one or two $(C_1—C_3)$alkyl, $(C_1—C_3)$alkoxy, halo, OH, benzyloxy, $CF_3$, $(C_1—C_3)$alkyl-O-$(C_1—C_3)$alkylene, phenoxy or di[$(C_1—C_3)$alkyl]amino$(C_1—C_3)$alkylene;

and pharmaceutically-acceptable salts thereof, possess valuable anti-ulcer properties.

A preferred group of pyrimidines of formula XX are those wherein R is hydrogen or $(C_1—C_3)$alkyl, $R^3$ is hydrogen, X is 0, Z is S, n is 2, A is $CH_2$ and B is pyridyl optionally substituted by hydroxy, methoxy or methyl; and their pharmaceutically-acceptable salts.

In the above formula the 4-pyrimidone or 4-pyrimidinethione ring has been written with the following structure (A):

A

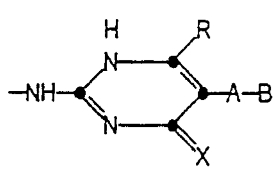

It should be recognized, however, that this structure is tautomeric with the following structures (B and C):

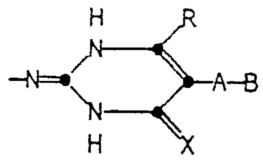

B

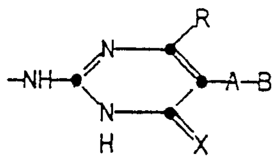

C

It is to be clearly understood that all such tautomeric equivalents are embraced by the present invention. Compounds according to structure A would be named systematically as 4(1H)-pyrimidones, those according to structure B as 4(1,3H)-pyrimidones, and those according to structure C as 4(3H)-pyrimidones. Again, regardless of which tautomer name is used to describe a given compound, the other tautomers are included within that name as forming part of the invention.

In like fashion, the pyrimidine rings below exhibit enol-keto tautomerism

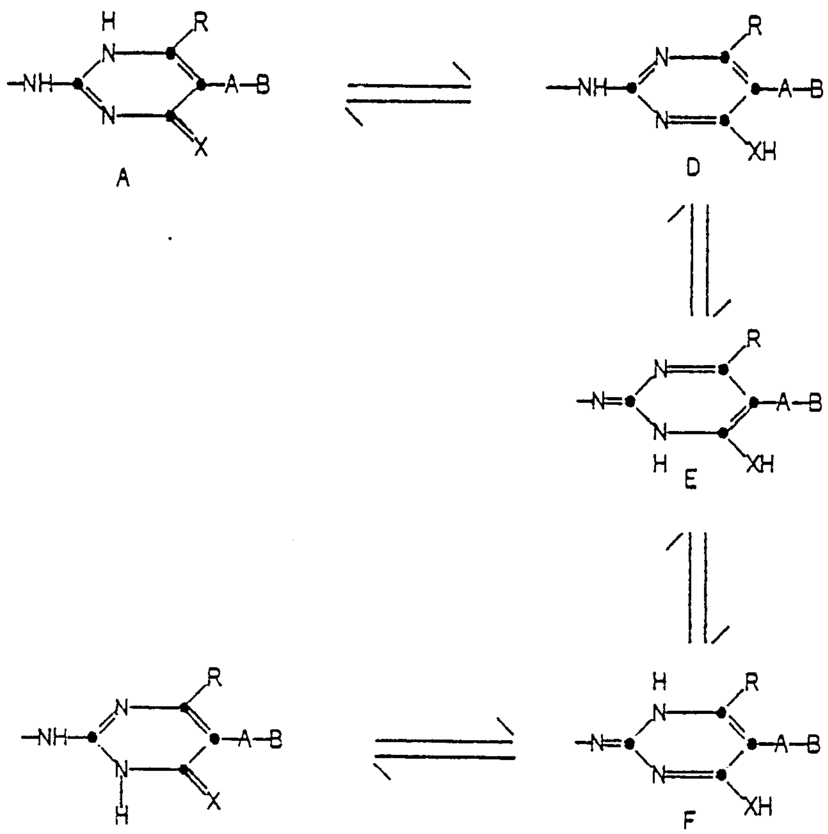

A D E C F

with a mono hydroxy or mercaptopyrimidine. Again, such tautomeric forms are included within the scope of the invention.

Studies of 4-pyrimidinone tautomerism indicate that structures A and C exist in approximately equal amounts, and that structure B is formed where the substituent at C—2 is an electron withdrawing group. Otherwise the oxo forms (A, B, C) predominate over the enol forms (D, E, F).

In Formula XX, the term $(C_1$—$C_3)$alkyl includes methyl, ethyl, n-propyl or isopropyl. Thus, the term $(C_1$—$C_3)$alkylphenyl would include o, m and p-tolyl, o, m, and p-ethylphenyl. Similarly, the term $(C_1$—$C_3)$alkoxyphenyl includes o, m, and p-anisyl, o, m, and p-ethoxyphenyl. The term halophenyl includes o, m, and p-chlorophenyl, bromophenyl, fluorophenyl and iodophenyl. Disubstituted phenyls such as 2,4-xylyl, 3,4-dichlorophenyl, 2-methyl-4-chlorophenyl and 3,4-dihydroxyphenyl are also included within the scope of the term "substituted phenyl".

The term $(C_3$—$C_6)$cycloalkyl includes cyclopropyl, cyclobutyl, cyclopentyl, cyclohexyl, methylcyclopentyl (all isomers), methylcyclobutyl and dimethylcyclobutyl.

The pyridyl groups which B can represent include 2-, 3- and 4-pyridyl.

Likewise, the alkyl, alkoxy, hydroxy, halo or amino substituents in the pyridyl or alicyclic aromatic ring may occupy any otherwise unoccupied portions in those rings.

A preferred group of radicals which B can represent includes 2, 3 and 4-pyridyl substituted with one or two methyl, methoxy or hydroxy groups such as 2-pyridyl, 3-pyridyl, 4-pyridyl, 6-methyl-3-pyridyl, 5-methyl-3-pyridyl, 2-methyl-3-pyridyl, 3-methoxy-4-pyridyl, 2-methoxy-4-pyridyl, and 5-(1,3-benzodioxolyl).

The pharmaceutically-acceptable salts of the compounds of this invention include acid-addition salts derived from inorganic acids such as: hydrochloric acid, nitric acid, phosphoric acid, sulfuric acid, hydrobromic acid, hydriodic acid, phosphorous acid and the like, as well as salts derived from organic acids such as aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate and naphthalene-2-sulfonate.

Compounds according to XX above can have three or more basic centers. These are a strongly basic amine group in the aminoalkyl group attached at position 2 of the thiazole ring, a less basic in the amine attached to the pyrimidone ring at C—2 and the basic ring nitrogen of the thiazole. A fourth basic group can also be present depending on the nature of the substituent "B"; for example, if B is pyridyl, the pyridyl nitrogen can form salts with strong acids. Thus, if a mineral acid is used, such as HCl or HBr, trihydrohalide salts can be prepared. Monohalide or dihalide salts are also preparable by titrating a solution of the base with acid and then evaporating the solution of the partially neutralized base to dryness. Weaker acids, such as some of the organic acids enumerated above, will form a salt only with the most basic center in the 2-dimethylaminomethylthiazole.

The compounds of this invention wherein Z is S or O — in other words, a heteroatom — are conveniently prepared from a (2-aminoalkyl-4-thiazolyl)methyl(heteroatom)alkylamine. The preparation of these starting materials is illustrated in Flow Chart A below using a compound in which the heteroatom is sulfur for exemplary purposes only.

Flow Chart A

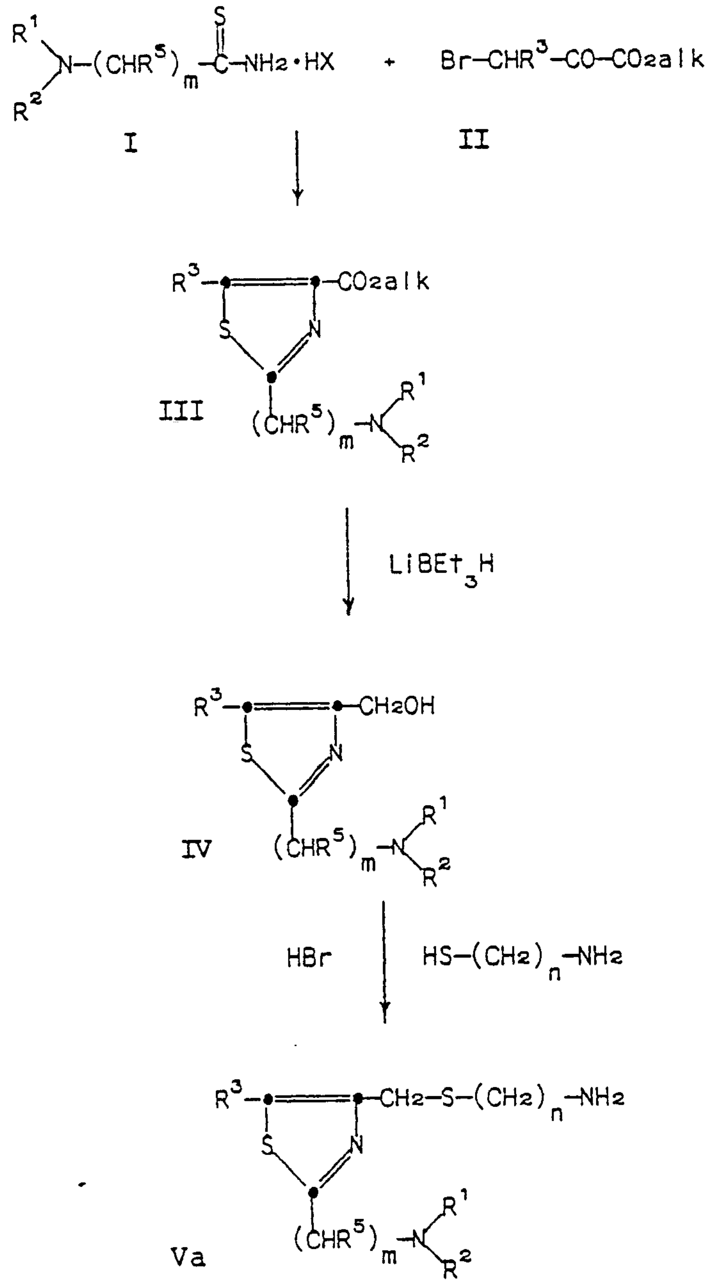


In the above Flow Chart, alk is conveniently methyl or ethyl and $R^1$, $R^2$, $R^3$, $R^5$, m and n have the same meaning as hereinabove.

In accordance with the above procedure, an acid addition salt of an aminoalkylthioacetamide (I) is reacted with a beta-bromo-alpha-ketoester (II) such as ethyl bromopyruvate ($R^3$=H) to yield an alkyl (methyl or ethyl) 2-(aminoalkyl)-4-thiazolecarboxylate (III). Reduction of this ester with a suitable hydride reducing agent such as lithium triethylborohydride, lithium aluminumhydride, sodium borohydride, diisobutylaluminumhydride and the like yields the corresponding hydroxymethyl compound (IV). Reaction of the 4-hydroxymethylthiazole with cysteamine or its higher homologue 3-mercaptopropylamine in the presence of acid yields directly a (2-aminoalkyl-4-thiazolylmethylthio)alkylamine (Va) optionally substituted with an alkyl group in the 5-position of the thiazole ring.

In the process indicated in Flow Chart A, in going from IV to Va, the hydroxymethyl group can be halogenated as with thionylchloride to yield a 4-chloromethylthiazole and the chlorinated compound in turn reacted with the sodium salt of the particular mercaptoalkylamine. In fact, any standard leaving group (a group labile to nucleophilic displacement) can be employed here in place of chloro in the chloromethyl side chain including for example p-tosyloxy, mesyloxy (methanesulfonyloxy), bromo, iodo and the like.

Alternatively, the 4-chloromethylthiazole hydrochloride (or other suitable acid addition salt) can be fused with a mercaptoalkylamine salt such as a hydrochloride salt to yield the desired primary amine (Va—Z=S).

If it is desired to prepare the side chain oxygen analogue of Va (Z=O), a process utilizing 2-chloroethylamine or 3-chloropropylamine to react with the 4-thiazolemethanol, under basic conditions, can be employed as well as can the analogous Williamson ether process using the sodium salt of the hydroxyalkylamine with a 4-thiazolylmethyl halide.

The compounds of this invention can be prepared by reacting an amine of formula V with a pyrimidone carrying in the two-position a group Q capable of undergoing nucleophilic displacement by a primary amine. The nature of such groups will be readily apparent to those skilled in the art. However, as examples thereof there may be mentioned groups such as nitroamino, ($C_1$—$C_3$)alkylthio, aralkylthio, typically benzylthio, halo, for instance, chloro or bromo, methylsulfinyl or methanesulfonyl. When Q is a nitroamino group a lower alkanol, such as ethanol, pyridine or acetonitrile can be used as the solvent, in which case it is preferred to heat the reaction mixture, conveniently at its reflux temperature. When Q is ($C_1$—$C_3$)alkylthio, the reaction can be carried out at an elevated temperature, with or without a solvent. However, if it is desired to use a solvent, suitable examples are DMSO, DMF, pyridine or lower alkanols, for example ethanol. In general, the condensation reaction may be effected at temperatures between 50 and 150°C, typically at the reflux temperature of the most appropriate reaction solvent.

FLOW CHART B

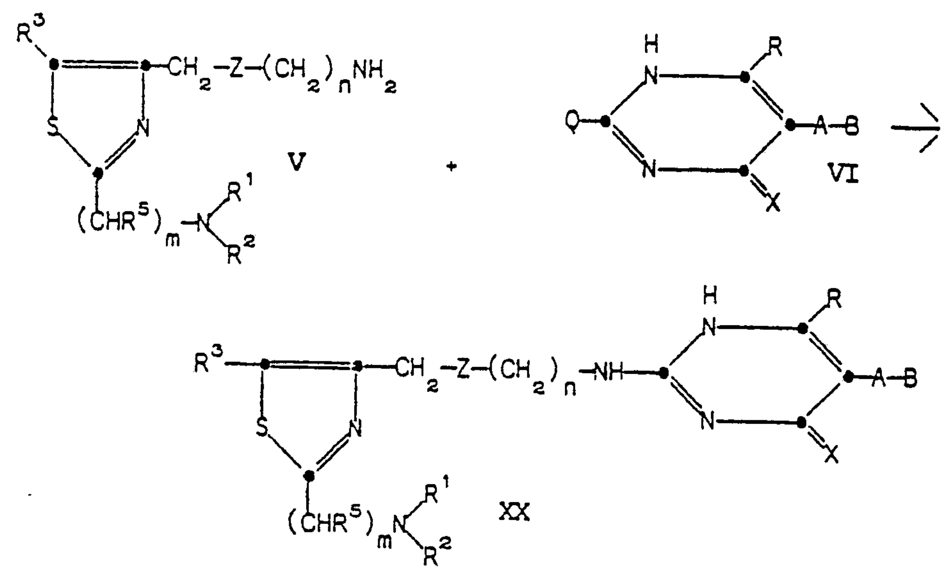

V

VI

XX

wherein R—$R^3$, $R^5$, n, m, x, Z, A and B have their previous significance and Q is a leaving group, except that $R^1$ and $R^2$ cannot both be H.

The preparation of amines of formula V is described in European Patent Specification No. 49,618 and the preparation of the pyrimidines of formula VI in, for instance, U.S. Patent No. 4,216,318.

Alternative processes are available for the preparation of the compounds of this invention. For example, the reaction scheme of Flow Chart B can be carried out with starting materials in which the roles are reversed; i.e., the leaving group is attached to the thiazole moiety and the reacting group (here OH or SH) is linked to the pyrimidone as in Flow Chart C.

FLOW CHART C

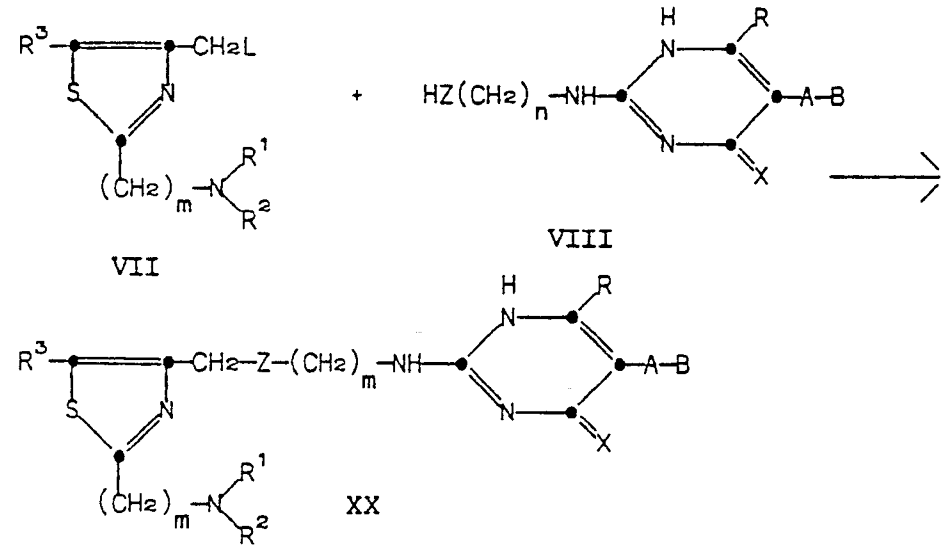

VII

VIII

XX

wherein R—$R^3$, X, Z, A, B, m and n have their previous significance and L is a leaving group such as OH, O-mesyl, O-tosyl, Cl, Br and the like. When L is OH, the reaction is carried out in the presence of a strong acid such as methanesulfonic acid or 12N hydrochloric acid. When L is a halogen (Cl or Br) or a halogen-like group (tosyloxy or mesyloxy), the reaction is carried out in the presence of a base such as the alkali metal (sodium) salt of a lower alkanol, and the same lower alkanol is conveniently used as a solvent. Optionally, when L is a halogen such as Cl or Br, the reaction can be carried out at an elevated temperature in the absence of a solvent by employing each reactant in the form of an acid addition salt, preferably as hydrochloride salts. The reaction is normally effected using reaction temperatures in the range from 50 to 150°C. It should be noted that the process of Flow Chart C cannot be used under acidic conditions if the group represented by "B" contains an ether function, such as a 6-methoxy-3-pyridyl group, since this ether group may be cleaved under the specified reaction conditions.

A second alternative synthetic procedure involves the reaction of a guanidine derivative of formula IX with a substituted α-acyl ester of formula X according to Flow Chart D.

FLOW CHART D

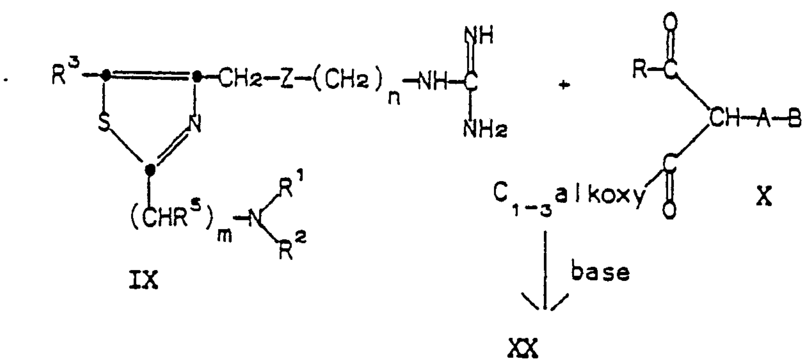


IX

X

XX

wherein R—$R^3$, $R^5$, m, n, A and B have their previous meanings and Z is S. The reaction of Flow Chart D may be effected by heating (50 to 150°C) the reactants in a mutual solvent, conveniently a ($C_1$—$C_3$)alkanol in which the ($C_1$—$C_3$)alkyl group is the same as that in the alkyl ester group of X, preferably in the presence of a base such as an alkali metal salt of the alkanol solvent. The N-2-(2-aminoalkyl-4-thiazolylthio)ethylguanidine (IX) can be prepared from the primary amine (V), by reaction with a salt of an alkylisothiourea

$NH_2$
(NH=C—S-alkyl)

such as a methylisothiouronium salt where the alkylthio group is a leaving group. Alternatively, the product IX can be prepared by guanylating the primary amine (V) with 3,5-dimethylpyrazole-1-carboxamidine.

In Flow Chart B, the pyrimidone or pyrimidonethione reactant VI has the structure

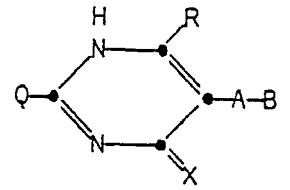

VI

where Q is a leaving group such as nitroamine, $(C_1—C_3)$alkylthio, benzylthio, halo, methylsulfinyl or methanesulfonyl. The pyrimidones (X=O) are conveniently prepared by a reaction similar to the ring closure reaction of Flow Chart D optionally followed by an alkylation reaction. For example, the α-acyl ester (X) can be reacted with thiourea to yield a 2-mercapto-5-substituted-4-pyrimidone of the formula

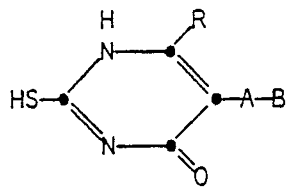

which intermediate can in turn be alkylated, as with an alkylhalide or dialkylsulfate, preferably in the presence of base to yield reactant VI of Flow Chart B wherein Q is a loweralkylthio $[(C_1—C_3)S]$ leaving group. The same compound can be prepared directly by reaction of the α-acylester (X) with an S-alkylisothiouronium salt preferably in the presence of base. When it is desired to prepare a pyrimidone (VI) in which Q is nitroamino, the above α-acyl ester is reacted with nitroguanidine in the presence of base, preferably a lower alkoxide in a lower alkanol solvent where the lower alkyl groups are the same.

Compounds wherein X is S in VI in Flow Chart B are prepared by acylating a pyrimidone (VI where Z is O) with a dilower alkylthiocarbamoyl chloride

$$[(C_1—C_3)alk]_2—N—\underset{\displaystyle Cl}{\underset{|}{C}}=S.$$

The O-dialkylthiocarbamate thus prepared is then thermally rearranged to yield the S-dialkylcarbamate. Hydrolysis yields the desired pyrimidinethione (VI where X is S).

The substituted pyrimidones starting materials (VIII) of Flow Chart C can be prepared from VI according to the following reaction scheme.

## FLOW CHART E

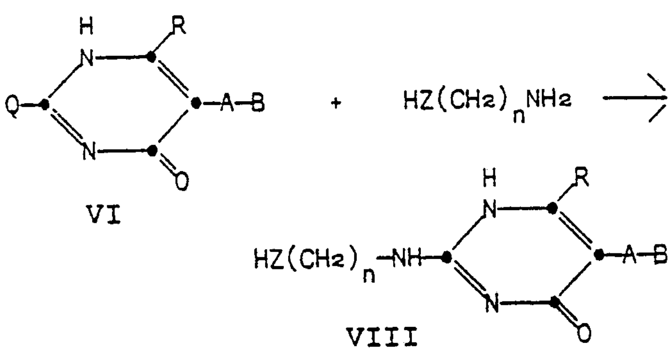

VI

VIII

wherein R, A, B, n and Q have their previously assigned meanings and Z is O or S. The reaction is carried out in an inert solvent such as a lower alkanol in the presence of an equimolar quantity of an alkali metal salt, preferably the sodium salt, of the lower alkanol.

The pyrimidone or pyrimidinethione moiety of the compounds of this invention (XX) is substituted in the 5-position with the grouping A—B where B may be an hydroxy substituted aryl or heteroaryl radical. These hydroxy substituted derivatives are conveniently prepared from the corresponding alkoxy substituted aryl or heteroaryl group (B) by hydrolysis for instance by reaction with conc. aqueous HCl or 48% aqueous HBr in ethanol or other equivalent solvent at an elevated temperature.

In the above reaction schemes, the aminoalkyl group present at position 2 of the thiazole ring has been shown as carrying through each of the reaction steps essentially unchanged from the starting material employed (I in Flow Chart A).

According to Flow Chart E the pyrimidone VI having a leaving group Q is reacted with cysteamine (or homocysteamine) or the corresponding hydroxy derivatives to yield a 2-(2-thio or oxy)ethylamino-5-substituted-4-pyrimidone (VIII). Reaction of this pyrimidone with a 2-aminoalkyl-4-thiazolylmethylchloride XI under reaction conditions sufficiently basic to produce an anion, illustratively a mercapto anion

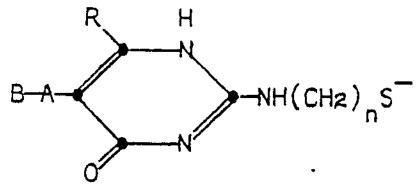

provides the compounds of this invention (XX) wherein Z is S or O (except that $R^1$ or $R^2$ may represent blocking groups).

Alternatively, the 2-(ω-mercaptoalkylamino)pyrimidone VIII as an acid addition salt can be fused with an acid addition salt of the 2-alkylamino-4-thiazolemethylchloride to yield the identical final product XXa.

The chloromethylthiazole VII where L is Cl is readily prepared by chlorination of the corresponding hydroxymethylthiazole (IV-Flow Chart A) with, for example, thionylchloride, $PCl_5$, $POCl_3$ etc.

The primary thiazolylalkyl amine reactants Vc (V wherein Z is $CH_2$ and n is 1, 2 or 3) can be prepared by the procedure illustrated in Flow Chart G below.

Flow Chart G

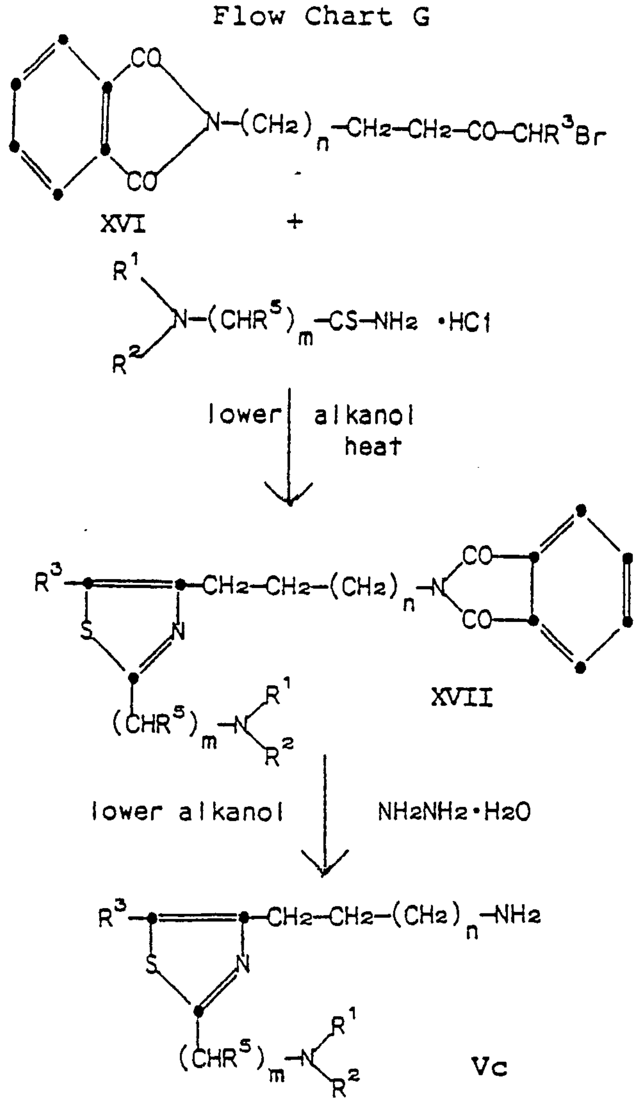


XVI + XVII Vc

wherein $R^1$, $R^2$, $R^3$, $R^5$, n and m have their previously assigned meaning.

According to flow chart G, an omega (phthalimido)alkyl halomethyl ketone (XVI) is reacted with a dialkylaminothioacetamide hydrochloride to produce a 2-aminoalkyl-5-permissibly-substituted-4-(omega-phthalimido)alkylthiazole (XVII). The protecting group is removed by hydrolysis with hydrazine hydrate to produce the 4-(omega-aminoalkyl)thiazole ($V_c$). Alkaline hydrolysis with an alkali metal hydroxide followed by treatment with a dilute hydrochloric acid can also be used. This primary amine product (Vc) corresponds to the starting material ($V_a$) produced by flow chart A and can undergo each of the reactions set forth in Flow Charts B—D to produce the compounds of this invention wherein Z in formula XX is $CH_2$.

This invention is further illustrated by the following specific examples.

## Example 1

Preparation of 2-[2-(2-dimethylaminomethyl-4-thiazolylmethylthio)ethyl]amino-5-(3-pyridyl)methyl-4-pyrimidone

A reaction mixture prepared from 1.24 g of 2-nitroamino-5-(3-pyridyl)methyl-4-pyrimidone, 1.14 g of 2-(2-dimethylaminomethyl-4-thiazolylmethylthio)ethylamine and 6 ml of anhydrous ethanol was refluxed for about 1 day under a nitrogen atmosphere. The volatile constituents were removed by evaporation to yield an oily residue. Water was added and the aqueous layer extracted with ether and with ethyl acetate. The organic extracts were combined, dried and evaporated to dryness to yield 2-[2-(2-dimethylaminomethyl-4-thiazolylmethylthio)ethyl]amino-5-(3-pyridyl)methyl-4-pyrimidone as a residue which was crystallized from ether. Recrystallization from an ethanol-ether solvent mixture yielded 2-[2-(2-dimethylaminomethyl-4-thiazolylmethylthio)ethyl]amino-5-(3-pyridyl)methyl-4-pyrimidone melting at 122—123°C.; yield = .68 g.

| | | | |
|---|---|---|---|
| Analysis Calculated: | C, 54.78; | H, 5.81; | N, 20.17 |
| Found: | C, 54.94; | H, 5.65; | N, 19.91 |

## Example 2

Preparation of 2-[2-(2-dimethylaminomethyl-4-thiazolylmethylthio)ethyl]amino-5-[5-(1,3-benzodioxolyl)]methyl-4-pyrimidone

Following the procedure of Example 1, 1.09 g of 2-(2-dimethylaminomethyl-4-thiazolylmethylthio)ethylamine and 1.40 g. of 2-nitroamino-5-[5-(1,3-benzodioxolyl)]methyl-4-pyrimidone were dissolved in 6 ml of anhydrous ethanol and the solution heated to reflux for about 1 day. The volatile constituents were removed by evaporation. The resulting residue was extracted with ethyl acetate and the ethyl acetate extract was washed with water, dried and the ethyl acetate removed therefrom by evaporation. Trituration with cyclohexane yielded crystalline material which was separated by filtration. Recrystallization from ethyl acetate yielded 0.38 g of 2-[2-(2-dimethylaminomethyl-4-thiazolylmethylthio)ethyl]amino-5-[5-(1,3-benzodioxolyl)]methyl-4-pyrimidone melting at about 110—111°C.

| | | | |
|---|---|---|---|
| Analysis Calculated: | C, 54.88; | H, 5.48; | N, 15.24 |
| Found: | C, 54.68; | H, 5.22; | N, 14.95. |

## Example 3

Preparation of 2-[2-(2-dimethylaminomethyl-4-thiazolylmethylthio)ethyl]amino-5-(6-methyl-3-pyridyl)-methyl-4-pyrimidone

Following the procedure of Example 1, 0.75 g of 2-(2-dimethylaminomethyl-4-thiazolylmethylthio)ethylamine and 0.78 g of 2-nitroamino-5-(6-methyl-3-pyridyl)methyl-4-pyrimidone were dissolved in 5 ml of anhydrous ethanol and the resulting solution refluxed for about 1 day. The solvents were removed by evaporation and the residue extracted with ethyl acetate. The ethyl acetate extract was washed with water and dried. Evaporation of the ethyl acetate yielded crystals of 2-[2-(2-dimethylaminomethyl-4-thiazolylmethylthio)ethyl]amino-5-(6-methyl-3-pyridyl)methyl-4-pyrimidone which melted at about 154—5°C after recrystallization from an ethyl acetate-ether solvent mixture; yield = 0.56 g.

| | | | |
|---|---|---|---|
| Analysis Calculated: | C, 55.79; | H, 6.09; | N, 19.52 |
| Found: | C, 56.02; | H, 6.06; | N, 19.50. |

## Example 4

Preparation of 2-[2-(2-dimethylaminomethyl-4-thiazolylmethylthio)ethyl]amino-5-(6-methoxy-3-pyridyl)-methyl-4-pyrimidone

Following the procedure of Example 1, 1.29 g of 2-(2-dimethylaminomethyl-4-thiazolylmethylthio)-ethylamine and 1.54 g of 2-nitroamino-5-(6-methoxy-3-pyridylmethyl)-4-pyrimidone were dissolved in 50 ml of ethanol and the resulting solution heated at reflux temperature for about 1 week. The solvent was removed by evaporation and the residue purified by gradient elution chromatography (silica-ethanol/ethyl acetate/ammonium hydroxide). Fractions containing the desired pyrimidone were combined and the solvents removed from the combined fractions. The resulting residue was crystallized from ethyl acetate to yield 2-[2-(2-dimethylaminomethyl-4-thiazolylmethylthio)ethyl]amino-5-(6-methoxy-3-pyridyl)methyl-4-pyrimidone melting at 137—8°C.

| | | | |
|---|---|---|---|
| Analysis Calculated: | C, 53.79; | H, 5.87; | N, 18.82 |
| Found: | C, 53.53; | H, 6.01; | N, 18.72. |

Example 5

Preparation of 2-[2-(2-dimethylaminomethyl-4-thiazolylmethylthio)ethyl]amino-5-(5,6-dimethyl-3-pyridyl)methyl-4-pyrimidone

Following the procedure of Example 1, 1.29 g of 2-(2-dimethylaminomethyl-4-thiazolylmethylthio)ethylamine and 1.29 g of 2-nitroamino-5-(5,6-dimethyl-3-pyridyl)methyl-4-pyrimidone were dissolved in 2.5 g of anhydrous ethanol and the mixture heated to refluxing temperature for 51 hours. The solvent was removed by evaporation and the residue purified by high pressure liquid gradient elution chromatography (silica-ethanol/ethyl acetate/ammonium hydroxide). Fractions containing 2-[2-(2-dimethylaminomethyl-4-thiazolylmethylthio)ethyl]amino-5-(5,6-dimethyl-3-pyridyl)methyl-4-pyrimidone were combined and the solvents removed from the combined fractions. An excess of ethanolic hydrogen chloride was added to the resulting residue. Evaporation of the ethanol yielded 2-[2-(2-dimethylaminomethyl-4-thiazolylmethylthio)ethyl]amino-5-(5,6-dimethyl-3-pyridyl)methyl-4-pyrimidone trihydrochloride melting at about 207—210°C after two-fold recrystallization from an ethanol/ethyl acetate solvent mixture; yield = 1.13 g.

| | | | |
|---|---|---|---|
| Analysis Calculated: | C, 45.54; | H, 5.64; | N, 5.17 |
| Found: | C, 45.29; | H, 5.39; | N, 5.21. |

Example 6

Preparation of 2-[2-(2-dimethylaminomethyl-4-thiazolylmethylthio)ethyl]amino-5-(6-hydroxy-3-pyridyl)methyl-4-pyrimidone

A solution was prepared from 0.92 g of 2-[2-(2-dimethylaminomethyl-4-thiazolylmethylthio)ethyl]-amino-5-(6-methoxy-3-pyridyl)methyl-4-pyrimidone (from Example 4). A mixture of 25 ml of ethanol and 25 ml of 2N aqueous hydrochloric acid was added. The solution was heated to refluxing temperature for about 4 days after which time it was made basic by the addition of 5N aqueous sodium hydroxide. The volatile constituents were removed by evaporation. The resulting residue was triturated with ethanol and filtered. The solvent was removed from the filtrate and the residue chromatographed using high pressure liquid gradient elution chromatography (silica-ethanol/ethyl acetate/ammonium hydroxide). Fractions containing 2-[2-(2-dimethylaminomethyl-4-thiazolylmethylthio)ethyl]amino-5-(6-hydroxy-3-pyridyl)-methyl-4-pyrimidone were combined and the solvent removed from the combined fractions. The residue was crystallized from a mixture of ethyl acetate and ether to yield 0.61 g of 2-[2-(2-dimethylaminomethyl-4-thiazolylmethylthio)ethyl]amino-5-(6-hydroxy-3-pyridyl)methyl-4-pyrimidone melting at about 125—128°C.

| | | | |
|---|---|---|---|
| Analysis Calculated: | C, 52.76; | H, 5.59; | N, 19.43 |
| Found: | C, 52.64; | H, 5.39; | N, 19.21. |

Example 7

Preparation of 2-[2-(2-dimethylaminomethyl-4-thiazolylmethylthio)ethyl]amino-5-(2-methoxy-4-pyridyl)-methyl-4-pyrimidone

Following the procedure of Example 1, a reaction mixture was prepared from 1.62 g of 2-(2-dimethylaminomethyl-4-thiazolylmethylthio)ethylamine and 1.94 g of 2-nitroamino-5-(2-methoxy-4-pyridyl)methyl-4-pyrimidone and 30 ml of ethanol. The reaction mixture was stirred and heated at refluxing temperature for about 42 hours. The volatile constituents were removed by evaporation and the residue purified by high pressure liquid gradient elution chromatography (silica-ethanol/ethyl acetate/ammonium hydroxide). Fractions containing 2-[2-(2-dimethylaminomethyl-4-thiazolylmethylthio)ethyl]amino-5-(2-methoxy-4-pyridyl)methyl-4-pyrimidone were combined and the solvent removed therefrom. Recrystallization of the resulting residue from a cyclohexane-ether solvent mixture gave 2-[2-(2-dimethylaminomethyl-4-thiazolylmethylthio)ethyl]amino-5-(2-methoxy-4-pyridyl)methyl-4-pyrimidone melting at 79—80°C.

| | | | |
|---|---|---|---|
| Analysis Calculated: | C, 53.79; | H, 5.87; | N, 18.82 |
| Found: | C, 53.53; | H, 5.71; | N, 18.64. |

Example 8

Preparation of 2-[2-(2-dimethylaminomethyl-4-thiazolylmethylthio)ethyl]amino-5-(3-pyridyl)methyl-6-methyl-4-pyrimidone

A reaction mixture was prepared from 1.16 g of 2-[2-dimethylaminomethyl-4-thiazolylmethylthio]-ethylamine, 1.31 g of 2-nitroamino-5-(3-pyridyl)methyl-6-methyl-4-pyrimidone and 25 ml of anhydrous ethanol. The reaction mixture was heated at reflux temperature for about 5 days after which time the ethanol was removed by evaporation. The residue was chromatographed using high pressure liquid gradient elution chromatography (silica-ethanol/ethyl acetate/ammonium hydroxide). Fractions containing 2-[2-(2-dimethylaminomethyl-4-thiazolylmethylthio)ethyl]amino-5-(3-pyridyl)methyl-6-methyl-4-pyrimidone were combined and the solvents removed from the combined fractions. 1.1 g of crystalline material was obtained which melted at about 130—131°C after recrystallization from a mixture of ethanol and ether; yield = 1.03 g. 2-[2-(2-dimethylaminomethyl-4-thiazolylmethylthio)ethyl]amino-5-(3-pyridyl)-methyl-6-methyl-4-pyrimidone thus prepared had the following analysis:

| | | | |
|---|---|---|---|
| Analysis Calculated: | C, 55.79; | H, 6.09; | N, 19.52 |
| Found: | C, 55.73; | H, 5.98; | N, 19.31. |

## Example 9

Preparation of 2-[2-(2-dimethylaminomethyl-4-thiazolylmethylthio)ethyl]amino-5-(2-hydroxy-4-pyridyl)methyl-4-pyrimidone

Following the procedure of Example 6, a solution prepared from 0.51 g of 2-[2-(2-dimethylaminomethyl-4-thiazolylmethylthio)ethyl]amino-5-(2-methoxy-4-pyridyl)methyl-4-pyrimidone (from Example 7) in 15 ml of 2N aqueous hydrochloric acid and 10 ml of ethanol was heated to refluxing temperature for about 5 days. Volatile constituents were then removed by evaporation and the resulting residue was dissolved in water. The acidic aqueous solution was neutralized with ammonium hydroxide. Water was removed by evaporation. The residue was extracted several times with ethanol. The ethanol was removed from the combined ethanol extracts by evaporation. The residue remaining was purified by high pressure liquid gradient elution chromatography (silica-ethanol/ethyl acetate/ammonium hydroxide). Fractions containing 2-[2-(2-dimethylaminomethyl-4-thiazolylmethylthio)ethyl]amino-5-(2-hydroxy-4-pyridyl)-methyl-4-pyrimidone were combined and the solvent removed therefrom by evaporation. Crystallization of the residue from ethyl acetate yielded 200 mg of 2-[2-(2-dimethylaminomethyl-4-thiazolylmethylthio)-ethyl]amino-5-(2-hydroxy-4-pyridyl)methyl-4-pyrimidone melting at 152—154°C having the following analysis.

| | | | |
|---|---|---|---|
| Analysis Calculated: | C, 52.76; | H, 5.59; | N, 19.43 |
| Found: | C, 52.50; | H, 5.49; | N, 19.22. |

## Example 10

Preparation of 2-[2-(2-dimethylaminomethyl-4-thiazolylmethylthio)ethyl]amino-5-(2-pyridyl)methyl-4-pyrimidone

Following the procedure of Example 1, a reaction mixture was prepared from 1.62 g of 2-(2-dimethylaminomethyl-4-thiazolylmethylthio)ethylamine, 1.73 g of 2-nitroamino-5-(2-pyridyl)methyl-4-pyrimidone and 20 ml of ethanol. The reaction mixture was heated at refluxing temperature for about 21 hours. The solvents were removed *in vacuo* and the residue purified by high pressure liquid gradient elution chromatography (silica-ethanol/ethyl acetate/ammonium hydroxide). Fractions containing 2-[2-(2-dimethylaminomethyl-4-thiazolylmethylthio)ethyl-amino-5-(2-pyridyl)methyl-4-pyrimidone (as determined by TLC) were combined and the solvents removed from the combined fractions by evaporation. The resulting crystalline residue was dissolved in ethanol and a slight excess of 5N aqueous hydrochloric acid added. The reaction mixture was evaporated to dryness and the resulting crystalline residue recrystallized from a mixture of methanol and ethanol to yield 1.2 g of 2-[2-(2-dimethylaminomethyl-4-thiazolylmethylthio)ethyl]amino-5-(2-pyridyl)methyl-4-pyrimidone trihydrochloride melting at 205—207°C.

| | | | |
|---|---|---|---|
| Analysis Calculated: | C, 43.39; | H, 5.17; | N, 15.98 |
| Found: | C, 43.11; | H, 5.21; | N, 16.01. |

## Example 11

Preparation of 2-[2-(2-dimethylaminomethyl-4-thiazolylmethylthio)ethyl]amino-5-(4-pyridyl)methyl-4-pyrimidone

Following the procedure of Example 1, 2-nitroamino-5-(4-pyridyl)methyl-4-pyrimidone was reacted with 2-(2-dimethylaminomethyl-4-thiazolylmethylthio)ethylamine to yield 2-[2-(2-dimethylaminomethyl-4-thiazolylmethylthio)ethyl]amino-5-(4-pyridyl)methyl-4-pyrimidone as a trihydrochloride salt melting at 200—202°C after recrystallization from a methanol/ethanol solvent mixture.

| | | | |
|---|---|---|---|
| Analysis Calculated: | C, 43.39; | H, 5.17; | N, 15.98 |
| Found: | C, 43.37; | H, 5.33; | N, 15.71. |

## Example 12

Preparation of 2-[2-(2-dimethylaminomethyl-4-thiazolylmethylthio)ethyl]amino-5-(3-pyridyl)methoxy-4-pyrimidone

A reaction mixture was prepared from 1.26 g of 2-(2-dimethylaminomethyl-4-thiazolylmethylthio)ethylamine and 1.36 g of 2-methylthio-5-(3-pyridyl)methoxy-4-pyrimidone in 20 ml of pyridine. The reaction mixture was heated to reflux temperature for about 2 days. The pyrimidine was then removed by evaporation *in vacuo* and the resulting residue subjected to high pressure liquid chromatography over silica. Fractions shown by tlc to contain the desired compound were combined and the combined fractions rechromatographed on a silica preparative plate using 3% ammoniated ethanol as the solvent. 2-[2-(2-Dimethylaminomethyl-4-thiazolylmethylthio)ethyl]amino-5-(3-pyridyl)methoxy-4-pyrimidone free base thus obtained was converted to the trihydrochloride salt by standard procedures. Recrystallization of the crude trihydrochloride salt from ethanol yielded 2-[2-(2-dimethylaminomethyl-4-thiazolylmethylthio)ethyl]-amino-5-(3-pyridyl)methoxy-4-pyrimidone trihydrochloride melting at 183—185°C.

| | | | |
|---|---|---|---|
| Analysis Calculated: | C, 42.11; | H, 5.02; | N, 15.51 |
| Found: | C, 42.35; | H, 5.11; | N, 15.71. |

Example 13

Preparation of 2-[2-(2-dimethylaminomethyl-4-thiazolylmethylthio)ethyl]amino-5-(3-pyridyl)methyl-4-pyrimidinethione

Following the procedure of Example 12, a mixture of 1.11 g of 2-(2-dimethylaminomethyl-4-thiazolylmethylthio)ethylamine and 1.24 g of 2-methylthio-5-(3-pyridyl)methyl-4-pyrimidinethione in 15 ml of pyridine was refluxed for 2 days. The desired reaction product was purified by gradient elution high pressure liquid chromatography over silica using an ethyl acetate/ethanol/ammonium hydroxide solvent system as the eluant. Fractions shown by tlc to contain 2-[2-(2-dimethylaminomethyl-4-thiazolylmethylthio)ethyl]amino-5-(3-pyridyl)methyl-4-pyrimidinethione free base were combined and the solvent removed by evaporation.

The free base was converted to the corresponding trihydrochloride salt by standard procedures. Recrystallization of the trihydrochloride salt from ethanol yielded 2-[2-(2-dimethylaminomethyl-4-thiazolylmethylthio)ethyl]amino-5-(3-pyridyl)methyl-4-pyrimidinethione trihydrochloride (0.8 g) melting at 187—189°C.

| | | | |
|---|---|---|---|
| Analysis Calculated: | C, 42.10; | H, 5.02; | N, 15.51 |
| Found: | C, 42.06; | H, 4.83; | N, 15.75. |

Example 14

Preparation of 2-[2-(2-Dimethylaminomethyl-4-thiazolylmethylthio)ethyl]amino-5-(6-methyl-3-pyridyl)oxy-4-pyrimidone

Following the procedure of Example 12, a mixture of 1.29 g of 2-(2-dimethylaminomethyl-4-thiazolylmethylthio)ethylamine and 1.38 g of 2-methylthio-5-(6-methyl-3-pyridyl)oxy-4-pyrimidone were heated to refluxing temperature for about 88 hours. After this time, the solvent was removed by evaporation *in vacuo* and the residue dissolved in ethanol. The ethanolic solution was evaporated to dryness. The residue was subjected to high pressure liquid chromatography over silica using a gradient elution technique with an ethyl acetate/ethanol/ammonium hydroxide solvent system as the eluant. Fractions shown by tlc to contain 2-[2-(2-dimethylaminomethyl-4-thiazolylmethylthio)ethyl]amino-5-(6-methyl-3-pyridyl)oxy-4-pyrimidone were combined and the solvent removed by evaporation. The resulting residue was rechromatographed on a silica preparative plate using 3% ammoniated ethanol as the solvent. 2-[2-(2-Dimethylaminomethyl-4-thiazolylmethylthio)ethyl]amino-5-(6-methyl-3-pyridyl)oxy-4-pyrimidone free base thus obtained was converted to the corresponding trihydrochloride salt by standard procedures. 2-[2-(2-Dimethylaminomethyl-4-thiazolylmethylthio)ethyl]amino-5-(6-methyl-3-pyridyl)oxy-4-pyrimidone trihydrochloride melted at about 216—220°C after recrystallization from a mixture of ethanol, ethyl acetate and ether. Yield = .36 g.

| | | | |
|---|---|---|---|
| Analysis Calculated: | C, 42.11; | H, 5.02; | N, 15.51 |
| Found: | C, 42.34; | H, 5.21; | N, 15.39. |

Example 15

Preparation of 2-[2-(2-dimethylaminomethyl-4-thiazolylmethylthio)ethyl]amino-5-[2-(3-pyridyl)ethyl]-4-pyrimidone

Following the procedure of Example 12, a mixture of 1.29 g of 2-(2-dimethylaminomethyl-4-thiazolylmethylthio)ethylamine and 1.37 g of 2-methylthio-5-[2-(3-pyridyl)ethyl]-4-pyrimidone in 25 ml of anhydrous pyridine was heated to refluxing temperature for about 4 days. The solvent was removed *in vacuo*. The residue was dissolved in ethanol and the ethanol evaporated. The residue was purified by high pressure liquid chromatography over silica using a gradient elution technique with an ethyl acetate/ethanol/ammonium hydroxide solvent system as the eluant. Fractions shown by tlc to contain the desired product were combined and the combined fractions rechromatographed on a silica preparative plate using 3% ammoniated ethanol as the solvent. 2-[2-(2-Dimethylaminomethyl-4-thiazolylmethylthio)ethyl]amino-5-[2-(3-pyridyl)ethyl]-4-pyrimidone free base thus prepared was converted by standard procedures to the trihydrochloride salt which melted at 226—228°C after recrystallization from an ethanol/ethyl acetate/ether solvent mixture. Yield = 1.0 g.

| | | | |
|---|---|---|---|
| Analysis Calculated: | C, 44.49; | H, 5.41; | N, 15.56 |
| Found: | C, 44.24; | H, 5.29; | N, 15.34. |

Similarly prepared using procedures described in the above Examples were:

Example 16

2-[2-(2-Dimethylaminomethyl-4-thiazolylmethylthio)ethyl]amino-5-(2-methyl-4-pyridyl)methyl-4-pyrimidone, trihydrochloride

from 2-(2-dimethylaminomethyl-4-thiazolylmethylthio)ethylamine and 2-methylthio-5-[2-(2-methyl-4-pyridyl)methyl-4-pyrimidone.

Yield 18%.

m.p. 191—3°C.

Example 17

2-[2-(2-Dimethylaminomethyl-4-thiazolylmethylthio)ethyl]amino-5-(5-methyl-3-pyridyl)methyl-4-pyrimidone, trihydrochloride hydrate

The title compound was prepared as a hydrate (3.5 $H_2O$) from 2-(2-dimethylaminomethyl-4-thiazolylmethylthio)ethylamine and 2-methylthio-5-(5-methyl-3-pyridyl)methyl-4-pyrimidone.

Yield 6%.

m.p. 80°C (foam), 220—40°C (dec.).

Example 18

2-[2-(2-Dimethylaminomethyl-4-thiazolylmethylthio)ethyl]amino-5-(2-methoxy-3-pyridyl)methyl-4-pyrimidone

The title product was similarly prepared from 2-(2-dimethylaminomethyl-4-thiazolylmethylthio)ethylamine and 2-nitroamino-5-(2-methoxy-3-pyridyl)methyl-4-pyrimidone.

Yield 44%.

m.p. (glass).

Example 19

2-[2-(2-dimethylaminomethyl-4-thiazolylmethylthio)ethyl]amino-5-(2-hydroxy-3-pyridyl)methyl-4-pyrimidone, dihydrochloride

The title compound was prepared by reacting 2-(2-dimethylaminomethyl-4-thiazolylmethylthio)ethylamine with 2-nitroamino-5-(2-methoxy-3-pyridyl)methyl-4-pyrimidone, followed by hydrolysis of the 2-methoxy substituent in the initial product to a 2-hydroxy group with hydrochloric acid.

Yield 11%.

m.p. 118—23°C (After recrystallization with EtOAc—$Et_2O$).

Starting materials useful in preparing the compounds of this invention can be prepared as described below in the following preparations.

Preparation 1

Preparation of Ethyl 2-Dimethylaminomethyl-4-thiazolecarboxylate

A reaction mixture was prepared containing 15.5 g of dimethylaminothioacetamide hydrochloride, 20.5 g of ethyl bromopyruvate and 100 ml of ethanol. The reaction mixture was heated to refluxing temperature for about four hours after which time the solvent was removed *in vacuo* in a rotary evaporator. The residue, containing ethyl 2-dimethylaminomethyl-4-thiazolecarboxylate formed in the above reaction, was dissolved in a mixture of ether and water. The aqueous layer was separated. The ether layer was extracted with an equal volume of water and then discarded. The two aqueous layers were combined and washed with ether. The ether layer was again discarded and the aqueous layer cooled to a temperature in the range of 0—5°C. Solid potassium carbonate was added until the aqueous layer gave a basic reaction to litmus. An oil separated comprising ethyl 2-dimethylaminomethyl-4-thiazolecarboxylate free base. The oily layer was extracted with ether and the ether extract separated and dried. The ether was removed by evaporation in vacuo. The resulting residue was purified by gradient high pressure liquid chromatography (silica ethyl acetate). Ethyl 2-dimethylaminomethyl-4-thiazolecarboxylate thus obtained had the following physical characteristics:

| | | | | |
|---|---|---|---|---|
| Analysis Calculated: | C, 50.45; | H, 6.59; | N, 13.07; | S, 14.96 |
| Found: | C, 50.13; | H, 6.39; | N, 12.89; | S, 15.04. |

The nmr spectrum in $CDCl_3$ (TMS internal standard) gave the following signals (δ): 1.43 (triplet, 3H), 2.40 (singlet, 6H), 3.87 (singlet, 2H), 4.47 (quartet, 2H), 8.20 (singlet, 1H).

Preparation 2

Preparation of 2-Dimethylaminomethyl-4-thiazolemethanol

A solution of 12.5 g of ethyl 2-dimethylaminomethyl-4-thiazolecarboxylate dissolved in about 35 ml of anhydrous tetrahydrofuran was prepared and then cooled to about 0°C under a nitrogen atmosphere. About 130 ml of a 1 molar solution of lithium triethylborohydride in THF was added in dropwise fashion while maintaining the temperature in the range 0—5°C. The reaction mixture was stirred for about two hours after which time 36 ml of 6N aqueous hydrochloric acid were added while maintaining the temperature in the range −3°C to 0°C. The volatile constituents were removed *in vacuo* on a rotary evaporator. Water was added to the resulting residue and again the volatile constituents were removed. Water was again added to the residue and the aqueous mixture extracted several times with ether. The ether extracts were separated and discarded. The aqueous solution was then chilled and made basic by the addition of solid potassium carbonate. The resulting alkaline aqueous mixture was extracted with ethyl acetate. 2-Dimethylaminomethyl-4-thiazolemethanol, being insoluble in the basic solution, separated and was extracted with several portions of ethyl acetate. The ethyl acetate extracts were combined, and the combined extracts washed with saturated aqueous sodium chloride and then dried. The ethyl acetate was removed by evaporation. The residue consisting of a brown oil weighing about 7.7 g comprised 2-dimethylaminomethyl-4-thiazolemethanol formed in the above reaction having the following physical characteristics.

Analysis Calculated: C, 48.81; H, 7.02; N, 15.26
Found: C, 48.71; H, 6.77; N, 15.85.

The nmr spectrum in $CDCl_3$ (TMS internal standard) gave the following signals (δ): 2.33 (singlet, 6H), 3.74 (singlet, 2H), 4.32 (singlet, 1H), 4.72 (singlet, 2H), 7.15 (singlet, 1H).

Boiling point = 102°C at 0.5 torr.

Alternatively, a mixture of 2.14 g of ethyl 2-dimethylaminomethyl-4-thiazolecarboxylate and 0.38 g of sodium borohydride in 20 ml of isopropanol was heated with stirring at reflux temperature for about 14 hours. The reaction mixture was cooled, and 2 ml of water were added carefully followed by 4 ml of 5N aqueous hydrochloric acid. The volatile constituents were removed by evaporation. Methanol (10 ml) was added and the mixture heated to refluxing temperature for about one hour. Methanol was removed by evaporation and the residual solids digested in 10 ml of isopropanol on the steam bath. The isopropanol solution was separated by decantation and the solids reextracted with 10 ml of isopropanol. The isopropanol solutions and extracts were combined and the combined solution filtered while hot to remove insoluble material. The filtrate was chilled and a crystalline solid appeared which separated and was recovered by filtration. Recrystallization of the filter cake from isopropanol gave 1.73 g of 2-dimethylaminomethyl-4-thiazolemethanol hydrochloride melting at 153—154°C.

Analysis Calculated: C, 40.28; H, 6.28; Cl, 16.99; N, 13.42
Found: C, 40.38; H, 5.04; Cl, 17.24; N, 13.12.

The methanols produced by the process of this example are readily converted to the corresponding thiazolemethyl chlorides according to the following procedure: A suspension was prepared from 1.05 grams of 2-dimethylaminomethyl-4-thiazolemethanol hydrochloride and 15 ml of chloroform. Thionyl chloride (2.50 g) was added and the resulting mixture was stirred at reflux temperature for about 2.75 hours. Any volatile constituents including excess thionyl chloride were removed by evaporation. The residue was suspended in chloroform and the chloroform removed by evaporation. The residue was then recrystallized from a methanol-ethyl acetate solvent mixture to yield 2-dimethylaminomethyl-4-thiazolylmethylchloride hydrochloride melting at 136—8°C.

Analysis Calculated: C, 37.01; H, 5.32; Cl, 31.21; N, 12.33
Found: C, 37.13; H, 5.06; Cl, 31.41; N, 12.36.

Preparation 3

Preparation of 2-(2-Dimethylaminomethyl-4-thiazolylmethylthio)ethylamine

A reaction mixture was prepared from 18.8 g of 2-dimethylaminomethyl-4-thiazolemethanol, 12.8 g of 2-aminoethanethiol hydrochloride (cysteamine hydrochloride) and 160 ml of 48% aqueous hydrobromic acid. The reaction mixture was stirred at about 100°C for about 11 hours. The volatile constituents were removed *in vacuo* on a rotary evaporator. Water was added and the volatile constituents again removed by evaporation. The resulting residue, comprising 2-(2-dimethylaminomethyl-4-thiazolylmethylthio)ethylamine trihydrobromide formed in the above reaction, was dissolved in ethanol. The ethanol was evaporated and the resulting residue again dissolved in ethanol. Evaporation of the ethanol yielded a hygroscopic residue which was recrystallized from a methanol-ethyl acetate solvent mixture. 2-(2-Dimethylaminomethyl-4-thiazolylmethylthio)ethylamine trihydrobromide thus prepared had the following physical characteristics:

Analysis Calculated: C, 22.80; H, 4.25; Br, 50.56; N, 8.86; S, 13.53
Found: C, 23.02; H, 4.31; Br, 50.64; N, 8.80; S, 13.60.

The nmr spectrum in $DMSOd_6$ (TMS internal standard) gave the following signals (δ): 2.55—3.2 (multiplet, 4H), 2.84 (singlet 6H), 3.92 (singlet, 2H), 4.74 (singlet, 2H), 7.2—7.7 (broad, 1H), 7.94 (singlet, 1H), 7.92 (broad, 3H), 10.22 (broad, 1H).

The above primary amine can be prepared by an alternate procedure involving the fusion of a 2-dialkylaminoalkyl-4-thiazolylmethylchloride acid addition salt with an acid addition salt of cysteamine (or homocysteamine). This alternate procedure is illustrated below.

2-Dimethylaminomethyl-4-thiazolylmethyl-chloride hydrochloride (1.92 g) and cysteamine hydrochloride (0.96 g) were intimately mixed and the mixture heated slowly under anhydrous conditions to about 100°C over a period of one hour. The reaction mixture was then heated in the range 104—110°C for a period of 6 hours at which time the reaction was substantially complete as determined by tlc [silica-95:5 ethanol-$NH_4OH$ (.88 sp. gr.)]. The reaction mixture was cooled and the cooled melt dissolved in a minimal amount of water. The solution was transferred to a rotary evaporator and the water removed. The resulting residue solidified and the solid was recrystallized from a methanol-ethyl acetate solvent mixture. Hygroscopic crystals of 2-(2-dimethylaminomethyl-4-thiazolylmethylthio)ethyl amine trihydrochloride thus produced melted at about 165—72°C with evolution of HCl.

Analysis calculated: C, 31.72; H, 5.91; Cl, 31.21; N, 12.33; S, 18.82.
Found: C, 31.63; H, 6.15; Cl, 31.34; N, 12.62; S, 18.63

In Flow Chart A above, compound I is a substituted aminothioacetamide hydrohalide of the structure

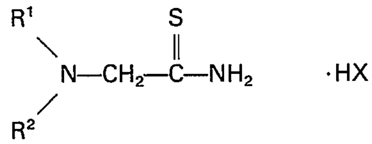

Where the substituting groups are methyl, the compounds are known and can be prepared by the method of *J. Org. Chem.,* (Russia), *6,* 884 (1970) in English.

One of the starting materials used in Flow Chart B is a 5-substituted-4-pyrimidone (VI). Such compounds, where the leaving group Q is a nitroamino group, are conveniently prepared by reacting, for example, nitroguanidine with a compound of the formula:

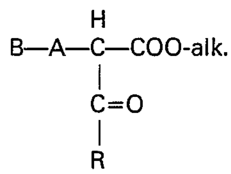

VIII

where alk, R, B and A have their previously assigned meaning. The esters of VIII in turn are prepared by acylating an ester of the formula B—A—$CH_2COOalk$ on the α-carbon. This series of reactions is illustrated in Flow Chart H below wherein R is H, B is a 3-pyridyl group and A is methylene.

Flow Chart H

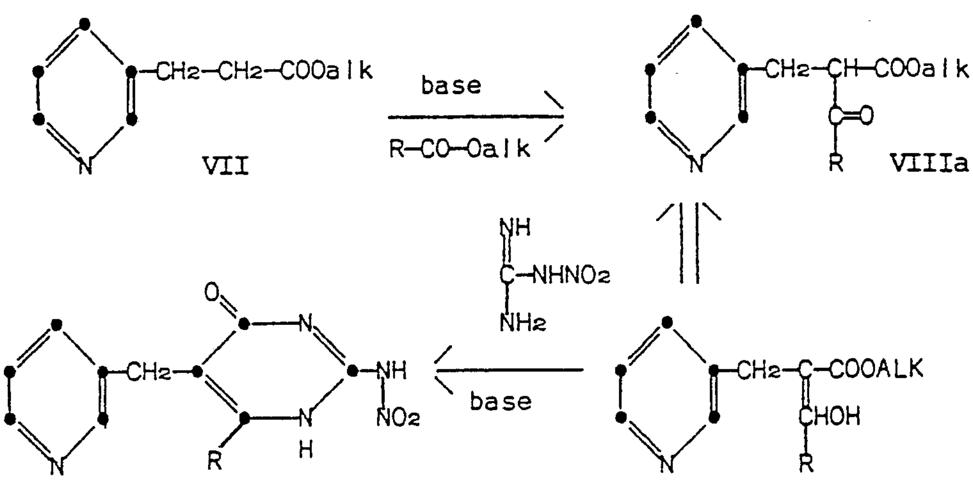


VII VIIIa

VIa VIIIb

The pyridyl substituted acetate can be prepared from a 3-pyridylacrylic acid ester or from a 3-pyridylpropiolic acid ester by reduction. It will be apparent that condensation of the α-acylpropionate with S-methyl-isothiourea (or S-benzylisothiourea) would provide a 4-pyrimidone having a methylthio (or benzylthio) leaving group (Q in formula VI). In addition, United States patent 4,216,318 discloses specific procedure for the preparation of compounds according to VI wherein Q is lower alkylthio, halo(Cl, Br) or benzylthio, A is alkylene, R is H or methyl and B is pyridyl, methylpyridyl, quinolyl, thienyl or thiazole.

The following additional references are particularly valuable for giving directions for the preparation of 5-substituted-4-pyrimidones or 4-pyrimidinethiones containing a leaving group in the 2-position corresponding to the following formula.

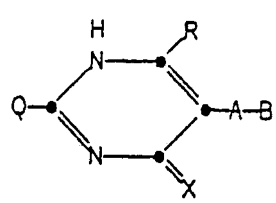

Belgian Patent 877,899, EPO Patents 3677, 7232, 51138, U.S. Patents 4,154,834, 4,234,588, 4,255,428.

The above references plus the following illustrative preparations can either supply directly the pyrimidone intermediates of the above formula or the procedures disclosed therein or in the voluminous literature associated with pyrimidine chemistry can be readily adapted by one skilled in the art to provide any desired pyrimidone.

Preparation 4

Preparation of 2-methylmercapto-5-(6-methyl-3-pyridyl)oxy-4-pyrimidone.

A suspension of 5.5 g of sodium hydride in 125 ml of anhydrous dimethylformamide was prepared. 25.1 g of 3-hydroxy-6-methylpyridine was added portionwise thereto. The consequent reaction mixture was stirred for about 1 hour and then cooled in an ice bath. 40.7 g of ethyl bromoacetate in 20 ml of ether were added thereto. The resulting reaction mixture was stirred at room temperature for about 3 days. A solid which precipitated was collected by filtration. The solvents were evaporated from the filtrate in vacuo, and the resulting residue dissolved in water. The aqueous solution was acidified by the addition of 19 ml of 12N aqueous hydrochloric acid. The acidic aqueous layer was extracted several times with ether. The ether extracts were discarded. The acidic aqueous layer was then made basic by the addition of 55 ml of 5N aqueous sodium hydroxide. An oil comprising ethyl (6-methyl-3-pyridyl)oxyacetate formed in the above reaction, separated and was extracted into ether. The ether extract was washed with water and then dried. The ether was removed therefrom by evaporation yielding 11.7 g of ethyl (6-methyl-3-pyridyl)oxyacetate as an oil.

Mass spectrum: m/e = 195

A suspension of 2.85 g of sodium hydride was prepared in 30 ml of 1,2-dimethoxyethane. The suspension was cooled and stirred while a mixture containing 10.8 g of ethyl formate and 18.6 g of ethyl (6-methyl-3-pyridyloxy)acetate was added thereto. The consequent reaction mixture was stirred for about 1 day and then poured over ice water. The reaction mixture was then extracted several times with ether. The ether extracts were discarded. The remaining aqueous layer was acidified to a pH in the range 6—7 with 2N aqueous sulfuric acid. The resulting neutral layer was extracted with ether and the ether extract separated and dried. Evaporation of the ether yielded 10.5 g of ethyl formyl-(6-methyl-3-pyridyloxy)acetate as a brown gum.

Mass spectrum: m/e = 223.

13.2 g of sodium were added to 400 ml of anhydrous ethanol. After all of the sodium had reacted to form sodium ethoxide, a solution containing 23.7 g of thiourea and 58.1 g of ethyl formyl-(6-methyl-3-pyridyloxy)acetate in 360 ml of ethanol was added. The consequent reaction mixture was heated to reflux temperature for about 16 hours and was then cooled to about 0°C. 44.4 g of methyl iodide were added thereto in dropwise fashion. The reaction mixture was stirred at room temperature for about an additional 18 hours after which time the volatile components were removed by evaporation. The residue remaining was dissolved in water. The pH of the resulting aqueous solution was adjusted to a pH in the range of 6—7 with 1N aqueous hydrochloric acid. A solid comprising 2-methylthio-5-(6-methyl-3-pyridyloxy)-4-pyrimidone precipitated and was separated by filtration. The filter cake was washed with water and ether. 34.3 g of 2-methylmercapto-5-(6-methyl-3-pyridyl)oxy-4-pyrimidone were obtained melting at 162—164°C.

Preparation 5

Preparation of 2-methylthio-5-[2-(3-pyridyl)-ethyl]-4-pyrimidone

A suspension of 2.53 g of sodium hydride in 35 ml of anhydrous dimethyl formamide was prepared. The suspension was cooled to about 0°C with stirring. Next, a mixture of 9.56 g of ethyl formate and 19 g of ethyl 4-(3-pyridyl)butyrate were added. The consequent reaction mixture was stirred at about 0°C for 1 additional hour and then at room temperature for about 3 days. The reaction mixture was acidified with 2N aqueous hydrochloric acid and the acidic aqueous solution evaporated to a very small volume in vacuo. The acidity of the solution was adjusted to pH = 6 by the addition of solid potassium carbonate. The neutral aqueous layer was then extracted with ether. The ether layer was separated and dried. Evaporation of the ether yielded 16.4 g of ethyl 2-formyl-4-(3-pyridyl)-butyrate (formed in the above reaction) as an orange oil.

Mass spectrum: m/e = 222 (p + 1)

About 3.75 g of sodium were added to 220 ml of anhydrous ethanol thus forming a solution of sodium ethoxide in ethanol. After all of the sodium had reacted, a mixture of 6.73 g of thiourea and 16.35 g of ethyl 2-formyl-4-(3-pyridyl)butyrate were added. This reaction mixture was heated to refluxing temperature for 21 hours after which time the solvent was removed by evaporation. The resulting residue was dissolved in water and the aqueous layer extracted successively with ether and chloroform. The pH of the aqueous layer was then adjusted to about 7 with 12N aqueous hydrochloric acid. A yellow solid comprising 5-[2-(3-pyridyl)ethyl]-2-thiouracil precipitated and was collected by filtration. The filter cake was washed with water and a small amount of ether to yield 10.5 g of the uracil melting at 254—258°C with decomposition.

Mass spectrum( m/e = 233

2.18 g of sodium were added to 130 ml of anhydrous ethanol thus forming a solution of sodium ethoxide in ethanol. 10 g of 5-[2-(3-pyridyl)ethyl]-2-thiouracil were added and the resulting mixture was stirred for about 1.5 hours (thus forming the sodium salt of the thiol group) and was then cooled to about 0°C. A solution of 7.1 g of methyl iodide and 5 ml of ethanol was next added in dropwise fashion to the

chilled reaction mixture with stirring. The stirring was continued for an additional 3.5 hours while maintaining the temperature at 0°C. The reaction mixture was then allowed to warm up to room temperature, at which temperature it was stirred for an additional hour. The solvent was removed by evaporation in vacuo. The resulting residue was dissolved in water and the aqueous solution neutralized with 10% aqueous hydrochloric acid. A yellow solid precipitated and was collected by filtration. The filter cake was washed with water plus a small quantity of ether. 9.0 g of 2-methylthio-5-[2-(3-pyridyl)ethyl]-4-pyrimidone melting at 191—192°C were obtained.

Following the above procedure, 3.03 g of sodium were suspended in anhydrous ether in a nitrogen atmosphere. 5.93 g of ethyl formate plus 14.1 g of ethyl 3-pyridylmethoxyacetate were added. The product of the reaction, ethyl formyl-(3-pyridylmethoxy)acetate as the sodium salt, was suspended in 175 ml of ethanol and 5.8 g of thiourea were added. The product of this reaction, 5-(3-pyridylmethoxy)-2-thiouracil, melted at 243—245°C; weight = 10.4 g. 9.9 g of the uracil were slurried in 80 ml of ethanol. A solution containing 1.78 g of sodium hydroxide and 38 ml of water was added followed by 4.86 g of methyl iodide. 1.46 g of 2-methylthio-5-(3-pyridyl)methoxy-4-pyrimidone were obtained melting at 186—187°C.

Preparation 6

Preparation of 2-nitroamino-6-methyl-5-(3-pyridyl)methyl-4-pyrimidone

A solution of sodium methoxide in methanol was prepared by adding 1.8 g of sodium to 75 ml of anhydrous methanol. 7.04 g of dried nitroguanidine were added thereto under a positive nitrogen atmosphere. This mixture was heated at reflux temperature for about one-half hour, after which time 15 g of ethyl 2-(3-pyridyl)methylacetoacetate (prepared according to the procedure of United States Patent 4,216,318) were added thereto and the resulting reaction mixture was heated at reflux temperature for about 19 hours. The volatile constituents were removed by evaporation and the resulting residue dissolved in water. The aqueous solution was extracted 4 times with chloroform and the chloroform extracts were discarded. The aqueous layer was then made slightly acid (pH = 6) by the addition of 5N aqueous hydrochloric acid. A solid comprising 2-nitroamino-6-methyl-5-(3-pyridyl)methyl-4-pyrimidone formed in the above reaction precipitated and was collected by filtration. The filter cake was washed with water and dried. 7.1 g of product melting at 231—233°C were obtained. Recrystallization of the filter cake from a mixture of dimethylformamide and methanol yielded 4.8 g of 2-nitroamino-6-methyl-5-(3-pyridyl)methyl-4-pyrimidone melting at 238—239°C.

Preparation 7

Preparation of 2-methylthio-5-(3-pyridyl)-methyl-4-pyrimidinethione

A suspension was prepared for 1.2 g of sodium hydride in 40 ml of anhydrous DMF. 11.7 g of 2-methylthio-5-(3-pyridyl)methyl-4-pyrimidone were added thereto under a positive nitrogen atmosphere. The reaction mixture was heated with stirring until all of the suspended hydride had disappeared. Next, 7.4 g of dimethylthiocarbamoyl chloride were added in portions while maintaining the reaction mixture at room temperature. After all of the carbamoyl chloride had been added, the reaction mixture was heated to 75°C and stirred at that temperature for 3 hours. The reaction mixture was then poured into 400 ml of 1% aqueous potassium hydroxide. o-[2-methylthio-5-(3-pyridyl)-methyl-4-pyrimidyl]dimethylthiocarbamate formed in the above reaction precipitated and was separated by filtration. The filter cake was washed with water and ether. Recrystallization of the filter cake from isopropanol yielded 3.22 g of O-[2-methylthio-5-(3-pyridyl)methyl-4-pyrimidyl]dimethylthiocarbamate melting at 122—123°C.

2.67 g of the above carbamate were heated with stirring at 180°C for one-half hour, and were then taken up in a mixture of 10 ml of methanol, and 10 ml of 1N aqueous sodium hydroxide. The water and methanol were removed by evaporation and the resulting residue dissolved in water. The cooled solution was neutralized with 10 ml of 1N aqueous hydrochloric acid.

2-Methylthio-5-(3-pyridyl)methyl-4-pyrimidinethione formed in the above reaction precipitated and separated by filtration. The filter cake was washed with water. The compound thus prepared melted at 211—213°C after drying.

| | | | |
|---|---|---|---|
| Analysis Calculated: | C, 52.98; | H, 4.45; | N, 16.85; |
| Found: | C, 52.74; | H, 4.16; | N, 17.14. |

Preparation 8

Preparation of 2-nitroamino-5-(4-pyridyl)-methyl-4-pyrimidone

About 1.77 g of sodium were dissolved in 75 ml of anhydrous methanol under a positive nitrogen atmosphere. The sodium methoxide in methanol solution was cooled and 7.28 g of dried nitroguanidine added thereto. The reaction mixture was heated to reflux temperature briefly after which time 14.5 g of ethyl 2-formyl-3-(4-pyridyl)propionate (prepared by the method of United States Patent 4,216,318) were added thereto. This reaction mixture was then heated to refluxing temperature for about 19 hours. Volatile constituents were removed by evaporation and water was added to the residue. The aqueous mixture was extracted 3 times with chloroform and the chloroform extracts discarded. The aqueous solution was then chilled to about 0°C and 15.4 ml of 5N aqueous hydrochloric acid added. 2-Nitroamino-5-(4-pyridyl)methyl-4-pyrimidone formed in the above reaction precipitated and the precipitate was collected by filtration. The filter cake was washed with water and dried. 9.9 g of product were obtained melting at about 228—229°C.

Following the above procedure, sodium methoxide and nitroguanidine were heated to form the sodium salt. 17.8 g of ethyl 2-formyl-3-(2-pyridyl)propionate (prepared as described in United States Patent 4,216,318) were added thereto. 2-Nitroamino-5-(2-pyridyl)methyl-4-pyrimidone was isolated and purified by the above procedure. The compound melted at about 195—197°C. (yield = 17.2 g).

The compounds of this invention are potent $H_2$ receptor antagonists and thus potential anti-ulcer agents. The relation of the $H_2$ receptors to mammalian gastric secretion is described in an article by Black et al. *Nature, 236,* 385 (1972).

The following assay for $H_2$ receptor blocking activity was employed. Female albino rats were treated with estrone 24 hours prior to the initiation of the experiment. The rats were sacrificed and the uterine horns removed therefrom and suspended at ambient temperature in isolated organ baths containing De Jalon's solution. After equilibrium, the uterine strips were exposed to 50 millimole aqueous potassium chloride, which produced a sustained contraction. When the uterus is so contracted, histamine produces a dose-dependent $H_2$ receptor-mediated relaxation. A control dose-response curve to histamine is constructed on each tissue. Following thorough washout of the histamine after obtaining the control dose-response curve, each antagonist (a compound of this invention) was added for 30 minutes at a concentration of $10^{-5}$ molar. The uterine strips were then contracted with aqueous potassium chloride in the presence of the antagonist and a second dose-response curve to histamine obtained. In the presence of a competitive antagonist, the dose-response curve to histamine was shifted in parallel to the right with no depression of the maximum relative to the control curve. The dose ratio (DR) is calculated for each concentration of antagonist by dividing the $ED_{50}$ of histamine in the presence of the competitive antagonist by the $ED_{50}$ for histamine. The dissociation constant ($K_B$) of the antagonist is calculated from the dose-ratios by the following equation:

$$K_B = [\text{antagonist}]/(DR—1)$$

Cimetidine was included as an internal standard.

Results of the above assay carried out on selected compounds of this invention are set forth in Table 1. In the table, column 1 gives the name of the compound under test and column 2, the -log$K_b$ value for that compound.

TABLE 1

| name | -log$k_b$ |
|---|---|
| 2-[2-(2-dimethylaminomethyl-4-thiazolylmethylthio)ethyl]amino-5-(3-pyridyl)methyl-4-pyrimidone | 8.96 |
| 2-[2-(2-dimethylaminomethyl-4-thiazolylmethylthio)ethyl]amino-5-[5-(1,3-benzodioxolyl)]methyl-4-pyrimidone | 8.36 |
| 2-[2-(2-dimethylaminomethyl-4-thiazolylmethylthio)ethyl]amino-5-(6-methyl-3-pyridyl)methyl-4-pyrimidone | 7.95 |
| 2-[2-(2-dimethylaminomethyl-4-thiazolylmethylthio)ethyl]amino-5-(4-pyridyl)methyl-4-pyrimidone | 7.89 |
| 2-[2-(2-dimethylaminomethyl-4-thiazolylmethylthio)ethyl]amino-5-(2-pyridyl)methyl-4-pyrimidone | 7.76 |

A second assay for $H_2$ receptor blocking activity employs the isolated bullfrog gastric mucosa — see Warrick and Lin, *Communications in Chem. Pathology and Pharmacology, 13,* 149 (1976). The assay is carried out as follows: The gastric mucosa of the bullfrog (*Rana catesbeiana*) is separated from the musculature of the stomach and placed between a pair of Ussing chambers made of lucite. The chambers are filled with frog Ringer solution and acid secretion is stimulated by addition of histamine to the serosal side of the mucosa at a final concentration of $10^{-5}$M. Acid output is automatically titrated to pH 4.5. After steady response to $10^{-5}$M of histamine is established, the antagonist (a compound of this invention) is added to the serosal chamber and the maximal inhibition by each concentration of the $H_2$-antagonist is

recorded. From the dose-response curve, the $ED_{50}$ of the drug is calculated. Results obtained with compounds of this invention in this assay are set forth in Table 2.

In Table 2, column 1 gives the substituent at 6 in the pyrimidone ring, column 2 the "B" group (see formula at the head of the Table), and column 3 the molar $ED_{50}$.

TABLE 2

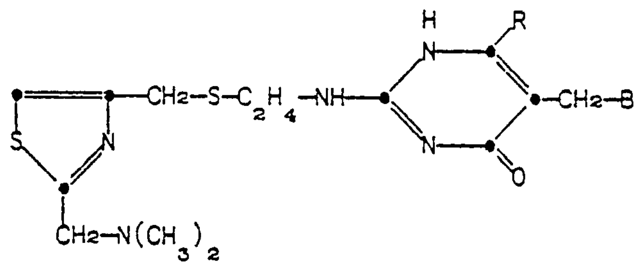

| R | B | $ED_{50}$ in moles |
|---|---|---|
| H | 3-pyridyl | $1.15 \times 10^{-7}$ |
| H | 6-methyl-3-pyridyl | $1.35 \times 10^{-6}$ |
| H | 6-methoxy-3-pyridyl | $1.26 \times 10^{-5}$ |
| H | 6-hydroxy-3-pyridyl | $2.52 \times 10^{-7}$ |
| H | 5-(1,3-benzodioxolyl) | $1.11 \times 10^{-5}$ |
| H | 4-pyridyl | $1.96 \times 10^{-7}$ |
| H | 2-pyridyl | $1.63 \times 10^{-7}$ |
| H | 5,6-dimethyl-3-pyridyl | $2.35 \times 10^{-6}$ |
| $CH_3$ | 3-pyridyl | $2.00 \times 10^{-6}$ |
| H | 2-methoxy-4-pyridyl | $3.00 \times 10^{-6}$ |
| H | 2-hydroxy-4-pyridyl | $6.03 \times 10^{-7}$ |

An *in vivo* assay for the antisecretory action of drugs on acid secretion utilizes gastric fistula dogs with a vagally innervated gastric fistula and vagally denervated Heidenthain pouch. A steady-state gastric secretion is produced by the iv infusion of histamine. The antisecretory drugs under test are given either intravenously by infusion over a 30 minute period or orally 75 min. prior to collection of gastric secretion from the fistula. In this assay, 2-[2-(2-dimethylaminomethyl-4-thiazolylmethylthio)ethyl]amino-5-(3-pyridyl)methyl-4-pyrimidone was 11X cimetidine by the iv route and 14X cimetidine orally. The 6-methyl-3-pyridyl analog was 14.4X cimetidine intravenously.

According to these and other tests, the compounds of this invention are more active than cimetidine, particularly when given orally. The compounds are also apparently much longer acting than cimetidine since recovery from an effective dose of 2-[2-(2-dimethylaminomethyl-4-thiazolylmethylthio)ethyl]amino-5-(3-pyridyl)methyl-4-pyrimidone is about 25% after 3—4 hours at which time cimetidine shows about a 50% recovering of acid secretions.

It is a further advantage of the compounds of this invention that they also possess H—1 histamine receptor inhibitory action, and thus can affect those physiological states which are produced by a mixture of $H_1$ and $H_2$ histamine receptor stimulation.

In utilizing the compounds of this invention as antisecretory agents, either the parenteral or oral route of administration may be employed. For oral dosage, a suitable quantity of a free base of this invention or a pharmaceutically-acceptable salt thereof formed with a non-toxic acid such as a hydrochloride salt is mixed with one or more conventional excipients such as starch and the mixture placed in telescoping gelatin capsules or compressed into tablets, each containing from 50—400 mg of active ingredients per dosage unit. The tablets may be scored if lower or divided dosages are to be used. For parenteral administration via an *iv* infusion, an isotonic solution of a salt is preferably employed although a soluble free base is also useful in isotonic preparations. However, the oral route of administration is preferred.

According to one aspect of the present invention there is provided a pharmaceutical formulation which comprises as an active ingredient a compound of formula XX, or a pharmaceutically-acceptable salt thereof, associated with one or more pharmaceutically-acceptable carriers or excipients therefor.

Because of the higher oral absorption and longer duration of action of the compounds of this invention, it is believed that oral administration of about 50—80 mg three to four times a day will suffice to control acid secretion in ulcer patients and thus alleviate ulcer symptoms. Generally, however, the compounds of this invention will be administered to humans orally in a daily dosage range of 140—800 mg. Smaller dosages at more frequent intervals may also be employed. The preferred oral dosage range is about 2—5 mg/kg/day of mammalian body weight, although a dosage range of from 1—10 mg/kg/day will include a great majority of patient regimes.

**Claims for the Contracting States: BE CH DE FR GB IT LI LU NL SE**

1. A compound of the formula XX:

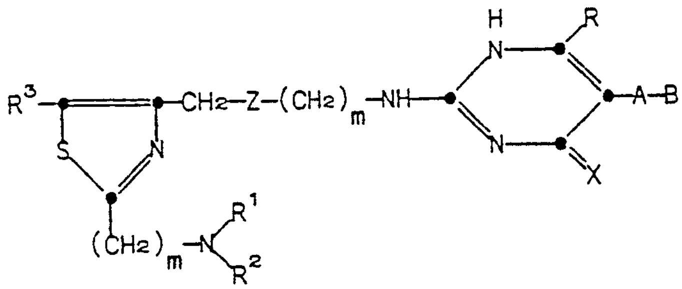

XX

wherein

R and $R^3$ independently represent hydrogen or $(C_1—C_3)$alkyl;

$R^1$ represents methyl;

$R^2$ represents methyl;

Z is O, S or $CH_2$;

X is S or O;

n is 2 or 3 when Z is O or S and n is 1, 2 or 3 when Z is $CH_2$;

$R^5$ is H;

m is 1;

A is $(C_1—C_5)$ alkylene or $(CH_2)_qX(CH_2)_p$ wherein q and p are individually 0, 1, 2 or 3 and the sum of q plus p is 0—4, and

B is H, $CH_3$, $(C_3—C_6)$cycloalkyl, a pyridyl group optionally substituted with one or two $(C_1—C_3)$alkyl, $(C_1—C_3)$ alkoxy, halo, hydroxy or amino groups;

naphthyl, (1,3-benzodioxolyl), 2,3-dihydro-1,4-benzodioxinyl, phenyl optionally substituted with one or two $(C_1—C_3)$alkyl, $(C_1—C_3)$alkoxy, halo, OH, benzyloxy, $CF_3$, $(C_1—C_3)$alkyl-O-$(C_1—C_3)$alkylene, phenoxy or di[$(C_1—C_3)$alkyl]amino$(C_1—C_3)$alkylene groups; or a pharmaceutically-acceptable salt thereof.

2. A compound of formula XX as claimed in claim 1, or a pharmaceutically-acceptable salt thereof, wherein R is hydrogen or $(C_1—C_3)$alkyl, $R^3$ is hydrogen, X is 0, Z is S, n is 2, A is $CH_2$ and B is pyridyl optionally substituted by hydroxy, methoxy or methyl.

3. 2-[2-(2-Dimethylaminomethyl-4-thiazolyl-methylthio)ethyl]amino-5-(3-pyridyl)methyl-4-pyrimidone.

4. 2-[2-(2-Dimethylaminomethyl-4-thiazolyl-methylthio)ethyl]amino-5-(6-methyl-3-pyridyl)methyl-4-pyrimidone.

5. 2-[2-(2-Dimethylaminomethyl-4-thiazolyl-methylthio)ethyl]amino-5-(2-methoxy-4-pyridyl)methyl-4-pyrimidone.

6. 2-[2-(2-Dimethylaminomethyl-4-thiazolyl-methylthio)ethyl]amino-5-(6-methoxy-3-pyridyl)methyl-4-pyrimidone.

7. A hydrochloride salt of a compound of formula XX as claimed in any one of claims 1 to 6.

8. A process for preparing a compound of formula XX, or a pharmaceutically-acceptable salt thereof, as claimed in any one of claims 1 to 7 which comprises:

(A) reacting an amine of formula V:

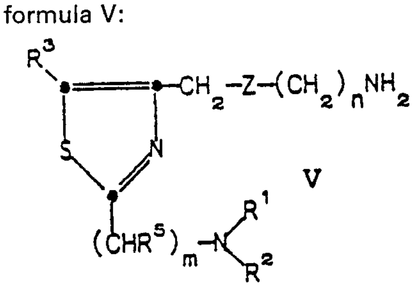

V

with a pyrimidine of formula:

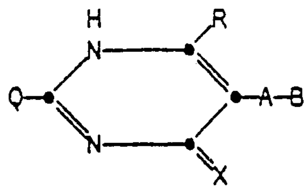

VI

where Q is a group capable of undergoing nucleophilic displacement by a primary amine;

(B) reacting a thiazole derivative of formula VII:

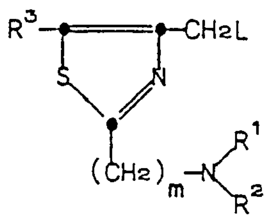

VII

with a compound of formula VIII;

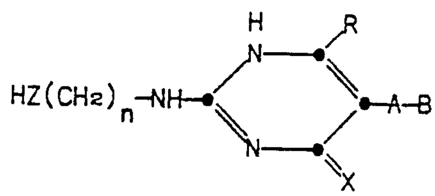

VIII

where Z is oxygen or sulfur; or

(C) reacting a guanidine derivative of formula IX:

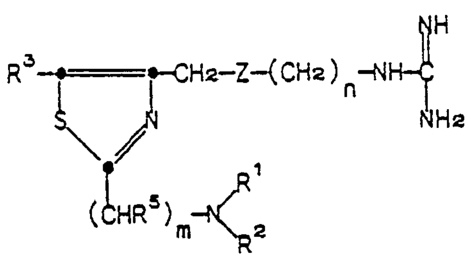

IX

with a substituted acyl ester of formula X:

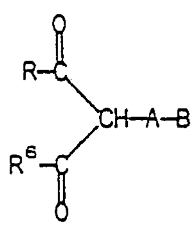

X

where $R^6$ is a ($C_1$—$C_3$) alkoxy group,

(D) optionally followed by hydrolysis of any alkoxy substituents in the B-moiety to the corresponding hydroxy substituents.

9. A compound of formula XX, or a pharmaceutically-acceptable salt thereof, as claimed in any one of claims 1 to 7, for use in ulcer chemotherapy.

10. A pharmaceutical formulation comprising as an active ingredient, a compound of formula XX, or a pharmaceutically-acceptable salt thereof, as claimed in any one of claims 1 to 7, associated with one or more pharmaceutically-acceptable carriers or excipients therefor.

**Claims for the Contracting State: AT**

1. A process for preparing a compound of the formula XX:

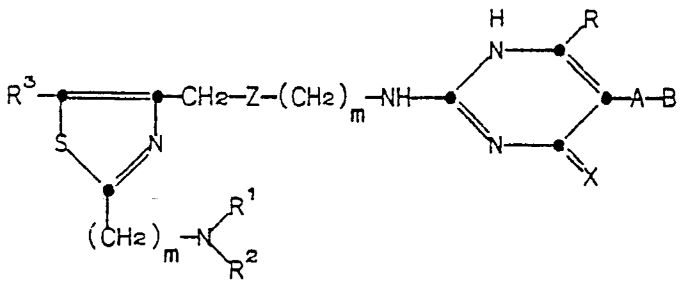

XX

wherein R and $R^3$ independently represent hydrogen or $(C_1—C_3)$alkyl;
$R^1$ represents methyl;
$R^2$ represents methyl;
Z is O, S or $CH_2$;
X is S or O;
n is 2 or 3 when Z is O or S and n is 1, 2 or 3 when Z is $CH_2$;
$R^5$ is H;
m is 1;
A is $(C_1—C_5)$ alkylene or $(CH_2)_qX(CH_2)_p$ wherein q and p are individually 0, 1, 2 or 3 and the sum of q plus p is 0—4, and
B is H, $CH_3$, $(C_3—C_6)$cycloalkyl, pyridyl group optionally substituted with one or two $(C_1—C_3)$alkyl, $(C_1—C_3)$ alkoxy, halo, hydroxy or amino groups;
naphthyl, (1,3-benzodioxolyl), 2,3-dihydro-1,4-benzodioxinyl, phenyl optionally substituted with one or two $(C_1—C_3)$alkyl, $(C_1—C_3)$alkoxy, halo, OH, benzyloxy, $CF_3$, $(C_1—C_3)$alkyl-O-$(C_1—C_3)$alkylene, phenoxy or di[($(C_1—C_3)$alkyl]amino$(C_1—C_3)$alkylene groups; or a pharmaceutically-acceptable salt thereof; which process comprises:

(A) reacting an amine of formula V:

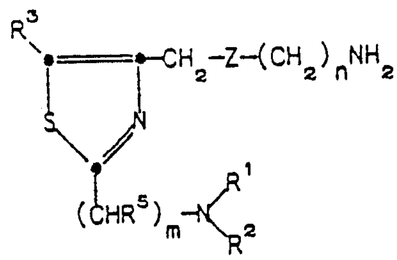

V

with a pyrimidine of formula:

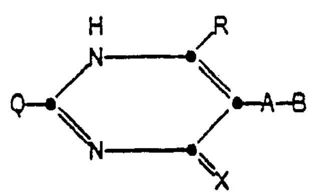

VI

where Q is a group capable of undergoing nucleophilic displacement by a primary amine;
(B) reacting a thiazole derivative of formula VII:

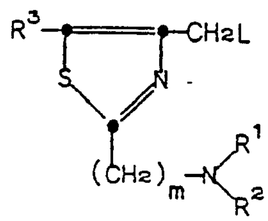

VII

with a compound of formula VIII:

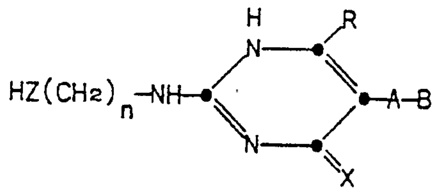

VIII

where Z is oxygen or sulfur; or

(C) reacting a guanidine derivative of formula IX:

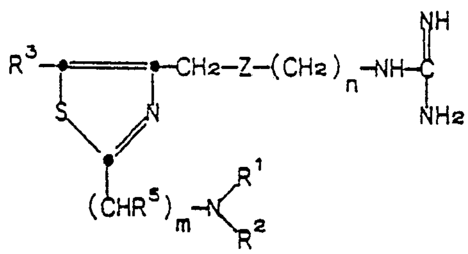

IX

with a substituted acyl ester of formula X:

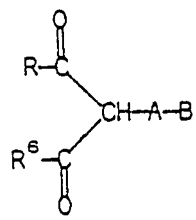

X

where $R^6$ is a $(C_1—C_3)$alkoxy group,

(D) optionally followed by hydrolysis of any alkoxy substituents in the B-moiety to the corresponding hydroxy substituents.

2. A process according to claim 1 for preparing a compound of formula XX, or a pharmaceutically-acceptable salt thereof, wherein R is hydrogen or $(C_1—C_3)$alkyl, $R^3$ is hydrogen, X is 0, Z is S, n is 2, A is $CH_2$ and B is pyridyl optionally substituted by hydroxy, methoxy or methyl.

3. A process according to claim 1 for preparing 2-[2-(2-dimethylaminomethyl-4-thiazolyl-methylthio)ethyl]amino-5-(3-pyridyl)methyl-4-pyrimidone.

4. A process according to claim 1 for preparing 2-[2-(2-dimethylaminomethyl-4-thiazolyl-methylthio)-ethyl]amino-5-(6-methyl-3-pyridyl)methyl-4-pyrimidone.

5. A process according to claim 1 for preparing 2-[2-(2-dimethylaminomethyl-4-thiazolyl-methylthio)-ethyl]amino-5-(2-methoxy-4-pyridyl)methyl-4-pyrimidone.

6. A process according to claim 1 for preparing 2-[2-(2-dimethylaminomethyl-4-thiazolyl-methylthio)-ethyl]amino-5-(6-methoxy-3-pyridyl)methyl-4-pyrimidone.

7. A compound of formula XX, or a pharmaceutically acceptable salt thereof, whenever prepared by a process according to any one of claims 1 to 6.

**Patentansprüche für die Vertragsstaaten: BE CH DE FR GB IT LI LU NL SE**

1. Pyrimidinderivate der Formel (XX)

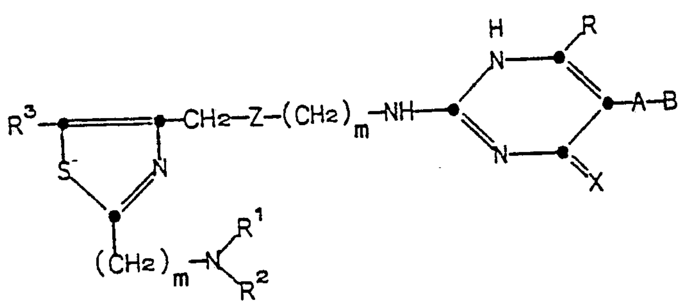

XX

worin

R und $R^3$ unabhängig Wasserstoff oder ($C_1$—$C_3$)-Alkyl sind,

$R^1$ Methyl bedeutet,

$R^2$ Methyl ist,

X für S oder O steht,

Z für O, S oder $CH_2$ steht,

n für 2 oder 3 steht, falls Z für O oder S steht, und n für 1, 2 oder 3 steht, falls Z für $CH_2$ steht,

$R^5$ für H steht,

m für 1 steht,

A für ($C_1$—$C_5$)-Alkylen oder $(CH_2)_qX(CH_2)_p$ steht, worin q und p einzeln 0, 1, 2 oder 3 bedeuten und die Summe aus q plus p für 0 bis 4 steht, und

B für H, $CH_3$, ($C_3$—$C_6$)-Cycloalkyl, eine Pyridylgruppe, die gegebenenfalls ein- oder zweifach durch ($C_1$—$C_3$)-Alkyl-, ($C_1$—$C_3$)-Alkoxy-, Halogen-, Hydroxy- oder Aminogruppen substituiert ist, Naphthyl, 1,3-Benzodioxoyl, 2,3-Dihydro-1,4-benzodioxinyl oder Phenyl steht, das gegebenenfalls ein- oder zweifach durch ($C_1$—$C_3$)-Alkyl, ($C_1$—$C_3$)-Alkoxy, Halogen, OH, Benzoyloxy, $CF_3$, ($C_1$—$C_3$)-Alkyl-O-($C_1$—$C_3$)-alkylen, Phenoxy oder Di[($C_1$—$C_3$)-alkyl]amino($C_1$—$C_3$)-alkylen substituiert ist, und die pharmazeutisch annehmbaren Salze hiervon.

2. Verbindung der Formel (XX) nach Anspruch 1 oder ein pharmazeutisch annehmbares Salz hiervon, dadurch gekennzeichnet, daß R Wasserstoff oder ($C_1$—$C_3$)-Alkyl ist, $R^3$ Wasserstoff bedeutet, X für O steht, Z für S steht, n für 2 steht, A für $CH_2$ steht und B pyridyl ist, das gegebenenfalls durch Hydroxy, Methoxy oder Methyl substituiert ist.

3. 2 - [2 - (2 - Dimethylaminomethyl - 4 - thiazolylmethylthio)ethyl]amino - 5 - (3 - pyridyl)methyl - 4 - pyrimidon.

4. 2 - [2 - (2 - Dimethylaminomethyl - 4 - thiazolylmethylthio)ethyl]amino - 5 - (6 - methyl - 3 - pyridyl)-methyl - 4 - pyrimidon.

5. 2 - [2 - (2 - Dimethylaminomethyl - 4 - thiazolylmethylthio)ethyl]amino - 5 - (2 - methoxy - 4 - pyridyl)methyl - 4 - pyrimidon.

6. 2 - [2 - (2 - Dimethylaminomethyl - 4 - thiazolylmethylthio)ethyl]amino - 5 - (6 - methoxy - 3 - pyridyl)methyl - 4 - pyrimidon.

7. Hydrochloridsalz einer Verbindung der Formel (XX) nach irgendeinem der Ansprüche 1 bis 6.

8. Verfahren zur Herstellung eines Pyrimidinderivats der Formel (XX) oder eines pharmazeutisch annehmbaren Salzes hiervon nach irgendeinem der Ansprüche 1 bis 7, dadurch gekennzeichnet, daß man

(A) ein Amin der Formel (V)

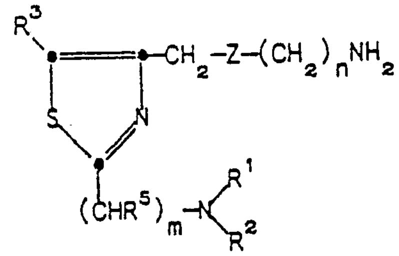

V

mit einem Pyridin der Formel (VI)

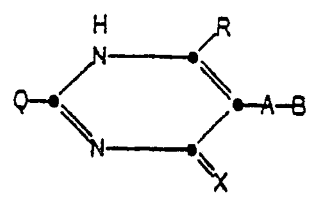

VI

worin Q eine zu einem nucleophilen Austausch durch ein primäres Amin befähigte Gruppe ist, umsetzt, oder

(B) ein Thiazolderivat der Formel (VII)

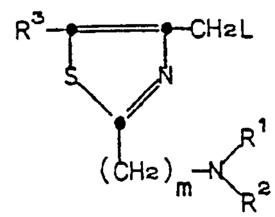

VII

mit einer Verbindung der Formel (VIII)

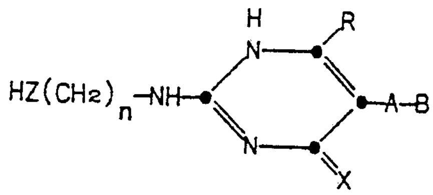

VIII

worin Z Sauerstoff oder Schwefel ist, zur Reaktion bringt oder

(C) ein Guanidinderivat der Formel (IX)

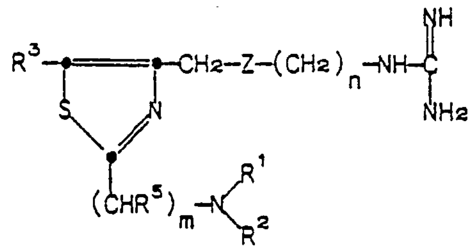

IX

mit einem substituierten Acylester der Formel (X)

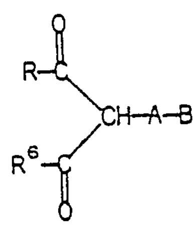

X

worin $R^6$ ein $(C_1—C_3)$-Alkoxygruppe ist, umsetzt und

(D) gegebenenfalls bei den erhaltenen Verbindungen vorhandene Alkoxysubstituenten im Rest B durch Hydrolyse in die entsprechenden Hydroxysubstituenten überführt.

9. Verwendung einer Verbindung der Formel (XX) oder eines pharmazeutisch annehmbaren Salzes hiervon gemäß irgendeinem der Ansprüche 1 bis 7 zur chemotherapeutischen Behandlung von Ulcera.

10. Pharmazeutische Formulierung, dadurch gekennzeichnet, daß sie eine Verbindung der Formel (XX) oder ein pharmazeutisch annehmbares Salz hiervon gemäß irgendeinem der Ansprüche 1 bis 7 als Wirkstoff in Kombination mit einem oder mehreren pharmazeutisch annehmbaren Trägern oder Hilfsstoffen hierfür enthält.

**Patentansprüche für den Vertragsstaat: AT**

1. Verfahren zur Herstellung eines Pyrimidinderivats der Formel (XX)

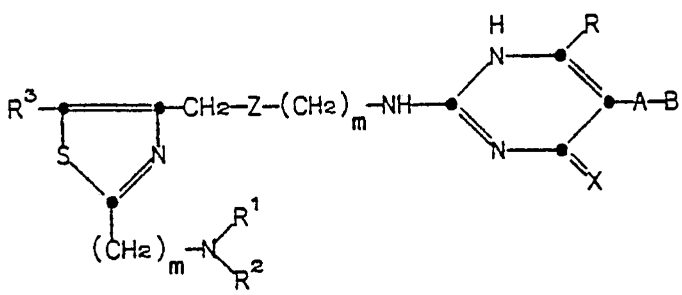

XX

worin

R und $R^3$ unabhängig Wasserstoff oder $(C_1—C_3)$-Alkyl sind,
$R^1$ Methyl bedeutet,
$R^2$ Methyl ist,
X für S oder O steht,
Z für O, S oder $CH_2$ steht,
n für 2 oder 3 steht, falls Z für O oder S steht, und n für 1, 2 oder 3 steht, falls Z für $CH_2$ steht,
$R^5$ für H steht,
m für 1 steht,

A für ($C_1$—$C_5$)-Alkylen oder $(CH_2)_qX(CH_2)_p$ steht, worin q und p einzeln 0, 1, 2 oder 3 bedeuten und die Summe aus q plus p für 0 bis 4 steht, und

B für H, $CH_3$, ($C_3$—$C_6$)-Cycloalkyl, eine Pyridylgruppe, die gegebenenfalls ein- oder zweifach durch ($C_1$—$C_3$)-Alkyl-, ($C_1$—$C_3$)-Alkoxy-, Halogen-, Hydroxy- oder Aminogruppen substituiert ist, Naphthyl, 1,3-Benzodioxoyl, 2,3-Dihydro-1,4-benzodioxinyl oder Phenyl steht, das gegebenenfalls ein- oder zweifach durch ($C_1$—$C_3$)-Alkyl, ($C_1$—$C_3$)-Alkoxy, Halogen, OH, Benzyloxy, $CF_3$, ($C_1$—$C_3$)-Alkyl-O-($C_1$—$C_3$)-alkylen, Phenoxy oder Di[($C_1$—$C_3$)-alkyl]amino($C_1$—$C_3$)-alkylen substituiert ist, oder eines pharmazeutisch annehmbaren Salzes hiervon, dadurch gekennzeichnet, daß man

(A) ein Amin der Formel (V)

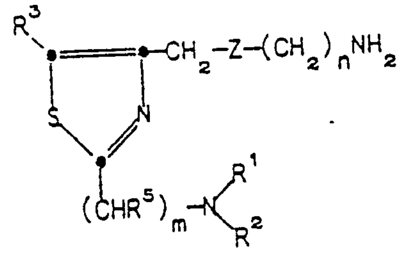

V

mit einem Pyridin der Formel (VI)

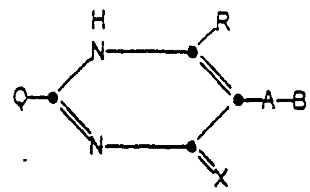

VI

worin Q eine zu einem nucleophilen Austausch durch ein primäres Amin befähigte Gruppe ist, umsetzt oder

(B) ein Thiazolderivat der Formel (VII)

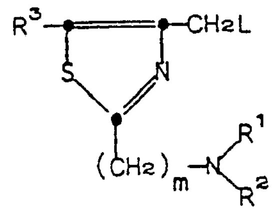

VII

mit einer Verbindung der Formel (VIII)

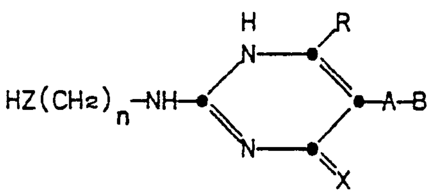

VIII

worin Z Sauerstoff oder Schwefel ist, zur Reaktion bringt oder

(C) ein Guanidinderivat der Formel (IX)

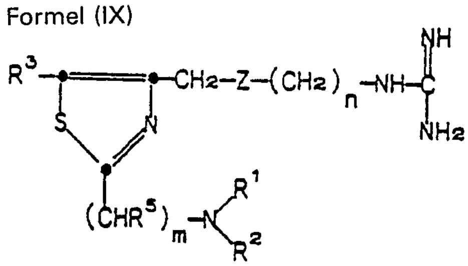

IX

mit einem substituierten Acylester der Formel (X)

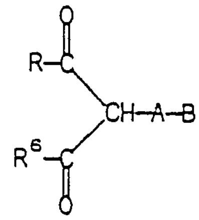

X

worin $R^6$ eine $(C_1—C_3)$-Alkoxygruppe ist, umsetzt und

(D) gegebenenfalls bei den erhaltenen Verbindungen vorhandene Alkoxysubstituenten im Rest B durch Hydrolyse in die entsprechenden Hydroxysubstituenten überführt.

2. Verfahren nach Anspruch 1, dadurch gekennzeichnet, daß man ein Verbindung der Formel (XX) oder ein pharmazeutisch annehmbares Salz hiervon herstellt, worin R Wasserstoff oder $(C_1—C_3)$-Alkyl ist, $R^3$ Wasserstoff bedeutet, X für O steht, Z für S steht, n für 2 steht, A für $CH_2$ steht und B pyridyl ist, das gegebenenfalls durch Hydroxy, Methoxy oder Methyl substituiert ist.

3. Verfahren nach Anspruch 1; dadurch gekennzeichnet, daß man 2 - [2 - (2 - Dimethylaminomethyl - 4 - thiazolylmethylthio)ethyl]amino - 5 - (3 - pyridyl)methyl - 4 - pyrimidon herstellt.

4. Verfahren nach Anspruch 1, dadurch gekennzeichnet, daß man 2 - [2 - (2 - Dimethylaminomethyl - 4 - thiazolylmethylthio)ethyl]amino - 5 - (6 - methyl - 3 - pyridyl)methyl - 4 - pyrimidon herstellt.

5. Verfahren nach Anspruch 1, dadurch gekennzeichnet, daß man 2 - [2 - (2 - Dimethylaminomethyl - 4 - thiazolylmethylthio)ethyl]amino - 5 - (2 - methoxy - 4 - pyridyl)methyl - 4 - pyrimidon herstellt.

6. Verfahren nach Anspruch 1, dadurch gekennzeichnet, daß man 2 - [2 - (2 - Dimethylaminomethyl - 4 - thiazolylmethylthio)ethyl]amino - 5 - (6 - methoxy - 3 - pyridyl)methyl - 4 - pyrimidon herstellt.

7. Pyrimidinderivat der Formel (XX) oder ein pharmazeutisch annehmbares Salz hiervon, dadurch gekennzeichnet, daß es nach einem Verfahren gemäß irgendeinem der Ansprüche 1 bis 6 hergestellt worden ist.

**Revendications pour les Etats contractants: BE CH DE FR GB IT LI LU NL SE**

1. Composé de formule XX:

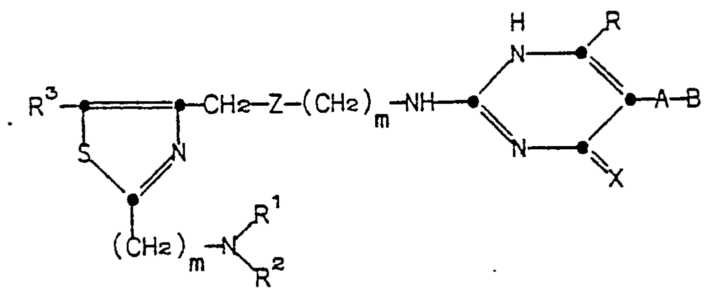

XX

dans laquelle

R et $R^3$ représentent chacun indépendamment l'un de l'autre un atome d'hydrogéne ou un groupe alkyle en $C_1—C_3$;

$R^1$ représente un groupe méthyle;

$R^2$ représente un groupe méthyle;

Z représente O, S ou $CH_2$;

X représente S ou O;

n est égal à 2 ou 3 lorsque Z représente O ou S et n est égal à 1, 2 ou 3 lorsque Z représente $CH_2$;

$R^5$ représente H;

m est égal à 1;

A représente un groupe alkylène en $C_1—C_5$ ou un groupe $(CH_2)_qX(CH_2)_p$ où q et p représentent chacun individuellement 0, 1, 2 ou 3, tandis que la somme de q plus p est de 0—4, et

B représente H, $CH_3$, un groupe cycloalkyle en $C_3—C_6$, un groupe pyridyle éventuellement substitué par un ou deux groupes alkyle en $C_1—C_3$, alcoxy en $C_1—C_3$, atomes d'halogènes, groupes hydroxyle ou amino; un groupe naphthyle, un groupe (1,3-benzodioxolyle), un groupe 2,3-dihydro-1,4-benzodioxinyle, un groupe phényle éventuellement substitué par un ou deux groupes alkyle en $C_1—C_3$, alcoxy en $C_1—C_3$, atomes d'halogènes, groupes OH, benzyloxy, $CF_3$, alkyl(en $C_1—C_3$)-O-(alkylène en $C_1—C_3$), phénoxy ou di-[alkyl en $C_1—C_3$]amino-alkylène en $C_1—C_3$; ou ses sels pharmaceutiquement acceptables.

2. Composé de formule XX selon la revendication 1 ou un de ses sels pharmaceutiquement acceptables, dans lequel R représente un atome d'hydrogène ou un groupe alkyle en $C_1—C_3$, $R^3$ représente un atome d'hydrogène, X représente O, Z représente S, n est égal à 2, A représente $CH_2$ et B représente un groupe pyridyle facultativement substitué par un groupe hydroxyle, un groupe méthoxy ou un groupe méthyle.

3. La 2 - [2 - (2 - diméthylaminométhyl - 4 - thiazolylméthylthio)éthyl]amino - 5 - (3 - pyridyl)méthyl - 4 - pyrimidone.

4. La 2 - [2 - (2 - diméthylaminométhyl - 4 - thiazolylméthylthio)éthyl]amino - 5 - (6 - méthyl - 3 - pyridyl)méthyl - 4 - pyrimidone.

5. La 2 - [2 - (2 - diméthylaminométhyl - 4 - thiazolylméthylthio)éthyl]amino - 5 - (2 - méthoxy - 4 - pyridyl)méthyl - 4 - pyrimidone.

6. La 2 - [2 - (2 - diméthylaminométhyl - 4 - thiazolylméthylthio)éthyl]amino - 5 - (6 - méthoxy - 3 - pyridyl)méthyl - 4 - pyrimidone.

7. Sel chlorhydrate d'un composé de formule XX selon l'une quelconque des revendications 1 à 6.

8. Procédé de préparation d'un composé de formule XX ou d'un de ses sels pharmaceutiquement acceptables selon l'une quelconque des revendications 1 à 7, caractérisé en ce qu'il comprend les étapes qui consistent à:

(A) faire réagir une amine de formule V:

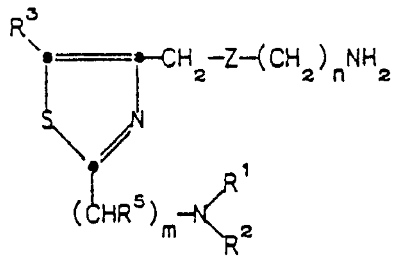

V

avec une pyrimidine de formule:

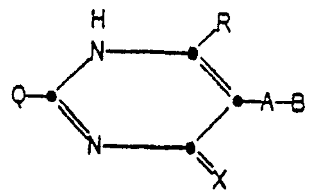

VI

dans laquelle Q représente un groupe capable de subir un déplacement nucléophile par une amine primaire;

(B) faire réagir un dérivé thiazole de formule VII:

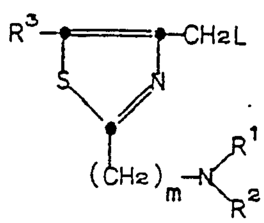

VII

avec un composé de formule VIII:

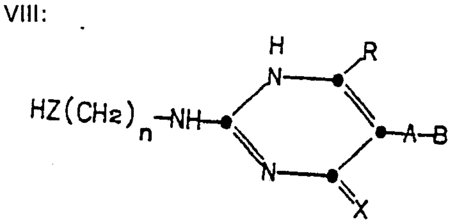

VIII

dans laquelle Z représente l'oxygène ou le soufre; ou

(C) faire réagir un dérivé de guanidine de formule IX:

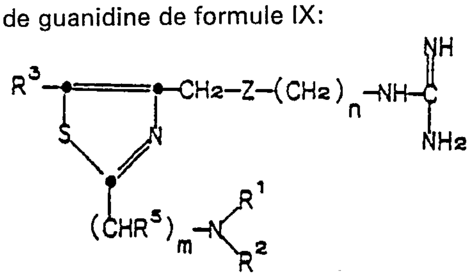

IX

avec un ester acylique substitué de formule X:

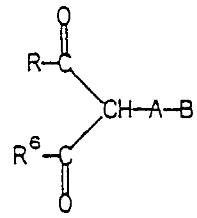

X

dans laquelle $R^6$ représente un groupe alcoxy en $C_1—C_3$;

(D) procéder ensuite facultativement à une hydrolyse de l'un ou l'autre substituant alcoxy dans la fraction B, pour obtenir les substituants hydroxyle correspondants.

9. Composé de formule XX ou un de ses sels pharmaceutiquement acceptables selon l'une quelconque des revendications 1 à 7, en vue de l'utiliser dans la chimiothérapie des ulcères.

10. Formulation pharmaceutique comprenant, comme ingrédient actif, un composé de formule XX, ou un de ses sels pharmaceutiquement acceptables selon l'une quelconque des revendications 1 à 7, en association avec un ou plusieurs supports ou excipients pharmaceutiquement acceptables pour ce composé ou ce sel.

**Revendications pour l'Etat contractant: AT**

1. Procédé de préparation d'un composé de formule XX:

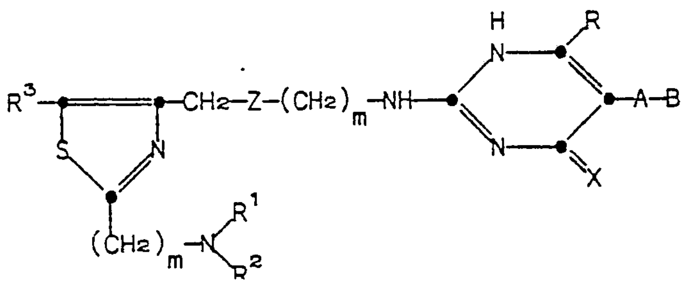

XX

dans laquelle

R et $R^3$ représentent chacun indépendamment l'un de l'autre un atome d'hydrogéne ou un groupe alkyle en $C_1—C_3$;

$R^1$ représente un groupe méthyle;

$R^2$ représente un groupe méthyle;

Z représente O, S ou $CH_2$;

X représente S ou O;

n est égal à 2 ou 3 lorsque Z représente O ou S, tandis que n est égal à 1, 2 ou 3 lorsque Z représente $CH_2$;

$R^5$ représente H;

m est égal à 1;

A représente un groupe alkylène en $C_1—C_5$ ou un groupe $(CH_2)_qX(CH_2)_p$ où q et p représentent chacun individuellement 0, 1, 2 ou 3, tandis que la somme de q plus p est égale à 0—4, et

B représente H, $CH_3$, un groupe cycloalkyle en $C_3—C_6$, un groupe pyridyle éventuellement substitué par un ou deux groupes alkyle en $C_1—C_3$, alcoxy en $C_1—C_3$, atomes d'halogènes, groupes hydroxyle ou amino;

un groupe naphthyle, un groupe (1,3-benzodioxolyle), un groupe 2,3-dihydro-1,4-benzodioxinyle, un groupe phényle éventuellement substitué par un ou deux groupes alkyle en $C_1—C_3$, alcoxy en $C_1—C_3$, atomes d'halogènes, groupes OH, benzyloxy, $CF_3$, alkyl(en $C_1—C_3$)-O-(alkylène en $C_1—C_3$), phénoxy ou di-[alkyl en $C_1—C_3$]amino-alkylène en $C_1—C_3$; ou d'un ses sels pharmaceutiquement acceptables, ce procédé comprenant les étapes consistant à:

(A) faire réagir une amine de formule V:

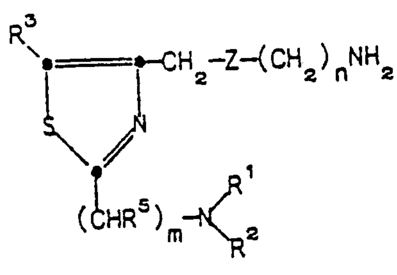

V

avec une pyrimidine de formule:

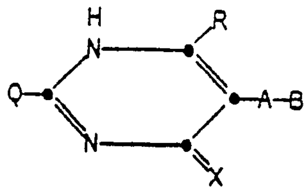

VI

où Q représente un groupe capable de subir un déplacement nucléophile par une amine primaire;

(B) faire réagir un dérivé de thiazole de formule VII:

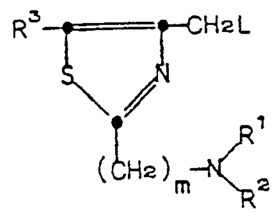

VII

avec un composé de formule VIII:

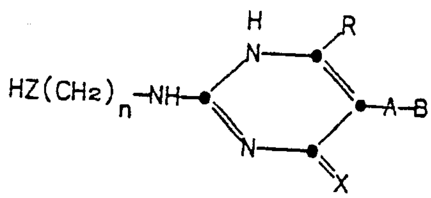

VIII

dans laquelle Z représente l'oxygène ou le soufre; ou

(C) faire réagir un dérivé de guanidine de formule IX:

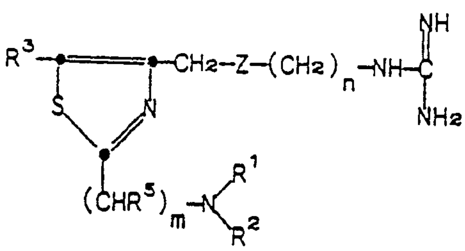

IX

avec un ester acylique substitué de formule X:

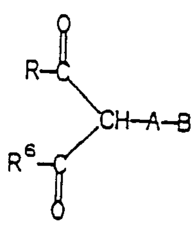

X

dans laquelle $R^6$ représente un groupe alcoxy en $C_1$—$C_3$;

(D) cette réaction étant facultativement suivie d'une hydrolyse de l'un ou l'autre substituant alcoxy dans la fraction B en substituants hydroxyle correspondants.

2. Procédé selon la revendication 1 en vue de préparer un composé de formule XX ou un de ses sels pharmaceutiquement acceptables, formule dans laquelle R représente un atome d'hydrogène ou un groupe alkyle en $C_1$—$C_3$, $R^3$ représente un atome d'hydrogène, X représente O, Z représente S, n est égal à 2, A représente $CH_2$ et B représente un groupe pyridyle éventuellement substitué par un groupe hydroxyle, un groupe méthoxy ou un groupe méthyle.

3. Procédé selon la revendication 1 pour la préparation de la 2 - [2 - (2 - diméthylaminométhyl - 4 - thiazolylméthylthio)éthyl]amino - 5 - (3 - pyridyl)méthyl - 4 - pyrimidone.

4. Procédé selon la revendication 1 pour la préparation de la 2 - [2 - (2 - diméthylaminométhyl - 4 - thiazolylméthylthio)éthyl]amino - 5 - (6 - méthyl - 3 - pyridyl)méthyl - 4 - pyrimidone.

5. Procédé selon la revendication 1 pour la préparation de la 2 - [2 - (2 - diméthylaminométhyl - 4 - thiazolylméthylthio)éthyl]amino - 5 - (2 - méthoxy - 4 - pyridyl)méthyl - 4 - pyrimidone.

6. Procédé selon la revendication 1 pour la préparation de la 2 - [2 - (2 - diméthylaminométhyl - 4 - thiazolylméthylthio)éthyl]amino - 5 - (6 - méthoxy - 3 - pyridyl)méthyl - 4 - pyrimidone.

7. Composé de formule XX ou un de ses sels pharmaceutiquement acceptables, préparé par un procédé selon l'une quelconque des revendications 1 à 6.